(19) Europäisches Patentamt / European Patent Office / Office européen des brevets

(11) **EP 4 038 360 B1**

(12) **EUROPÄISCHE PATENTSCHRIFT**

(45) Veröffentlichungstag und Bekanntmachung des Hinweises auf die Patenterteilung:
**29.10.2025 Patentblatt 2025/44**

(21) Anmeldenummer: **20785732.7**

(22) Anmeldetag: **30.09.2020**

(51) Internationale Patentklassifikation (IPC):
**G01N 1/30** (2006.01)   **A61P 43/00** (2006.01)
**G01N 33/68** (2006.01)   **G01N 33/94** (2006.01)
**C12Q 1/6883** (2018.01)

(52) Gemeinsame Patentklassifikation (CPC):
**G01N 1/30; A61P 43/00; G01N 33/9453;**
C12Q 1/6883; C12Q 2600/106; C12Q 2600/154;
G01N 2800/323; G01N 2800/52; G01N 2800/7052

(86) Internationale Anmeldenummer:
**PCT/EP2020/077401**

(87) Internationale Veröffentlichungsnummer:
**WO 2021/064040 (08.04.2021 Gazette 2021/14)**

(54) **ADJUVANTE ANTIFIBROTISCHE THERAPIE EINER SUBPOPULATION VON PATIENTEN NACH TAVI**

AJUVANT ANTIFIBROTIC THERAPY OF A SUBPOPULATION OF PATIENTS AFTER TAVI

THÉRAPIE ANTIFIBROTIQUE AJUVANT D'UNE SOUS-POPULATION DE PATIENTS APRES TAVI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.10.2019 DE 102019126517**

(43) Veröffentlichungstag der Anmeldung:
**10.08.2022 Patentblatt 2022/32**

(73) Patentinhaber: **Georg-August-Universität Göttingen Stiftung Öffentlichen Rechts, Universitätsmedizin 37075 Göttingen (DE)**

(72) Erfinder:
• HASENFUSS, Gerd
  37077 Göttingen (DE)
• PULS, Miriam
  37124 Rosdorf (DE)
• ZEISBERG, Elisabeth
  37073 Göttingen (DE)
• ZEISBERG, Michael
  37073 Göttingen (DE)
• TOISCHER, Karl
  37075 Göttingen (DE)

• TAMPE, Björn
  37077 Göttingen (DE)
• JACOBSHAGEN, Claudius
  76227 Karlsruhe (DE)

(74) Vertreter: **Wichmann, Hendrik Wuesthoff & Wuesthoff Patentanwälte und Rechtsanwalt PartG mbB Schweigerstraße 2 81541 München (DE)**

(56) Entgegenhaltungen:
• DAINIUS DAUNORAVICIUS ET AL: "Quantification of myocardial fibrosis by digital image analysis and interactive stereology", DIAGNOSTIC PATHOLOGY, BIOMED CENTRAL LTD, LO, vol. 9, no. 1, 9 June 2014 (2014-06-09), pages 114, XP021189567, ISSN: 1746-1596, DOI: 10.1186/1746-1596-9-114

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **SCHWARZ F ET AL: "Correlation between myocardial structure and diastolic properties of the heart in chronic aortic valve disease: Effects of corrective surgery", AMERICAN JOURNAL OF CARDIOLOGY, CAHNERS PUBLISHING CO., NEWTON, MA, US, vol. 42, no. 6, 1 December 1978 (1978-12-01), pages 895 - 903, XP026365035, ISSN: 0002-9149, [retrieved on 19781201]**
- **SAITO S ET AL: "Cardiac fibrosis and cellular hypertrophy decrease the degree of reverse remodeling and improvement in cardiac function during left ventricular assist", JOURNAL OF HEART AND LUNG TRANSPLANTATION, ELSEVIER, AMSTERDAM, NL, vol. 29, no. 6, 1 June 2010 (2010-06-01), pages 672 - 679, XP027060524, ISSN: 1053-2498, [retrieved on 20100225]**
- **OKADA R ET AL: "Relation between coronary stenosis and myocardial lesions determined by a semiquantitative approach to myocardial fibrosis and hypertrophy due to ischemia", AMERICAN JOURNAL OF CARDIOLOGY, CAHNERS PUBLISHING CO., NEWTON, MA, US, vol. 63, no. 10, 7 March 1989 (1989-03-07), pages E2 - E6, XP023208355, ISSN: 0002-9149, [retrieved on 19890307], DOI: 10.1016/ 0002-9149(89)90222-1**

**Beschreibung**

Die vorliegende Erfindung betrifft eine Kombination von Spironolacton mit Hydralazin und/oder Dihydralazin als Anti-Fibrose-Mittel zur Anwendung bei der Behandlung und/oder Prävention von Patienten mit Aortenstenose, die einer Katheter-basierten Aortenklappenimplantation (TAVI) unterzogen werden, oder der Nachbehandlung von Patienten mit Aortenstenose, die einer Katheter-basierten Aortenklappenimplantation unterzogen wurden. Diese Patienten mit Aortenstenose sind dadurch gekennzeichnet, dass sie eine myokardiale Fibrose aufweisen, die gleich oder höher als ein klinisch signifikanter Schwellenwert der Querschnittsfläche des Myokards ist. Ziel der Behandlung, Prävention bzw. Nachbehandlung ist es, die 1-Jahres-Sterblichkeit nach TAVI bei dieser Patientengruppe zu senken.

**HINTERGRUND DER ERFINDUNG**

Die Aortenklappenstenose ist die häufigste Herzklappenerkrankung in Deutschland und Europa. Die Häufigkeit nimmt mit zunehmendem Lebensalter zu. Sie beträgt in der Altersgruppe der über 80jährigen 10 %. In 2017 wurden in Deutschland 28763 Patienten mit Aortenklappenstenose durch TAVI (17956) oder operativen Klappenersatz behandelt (Deutscher Herzbericht 2018). Im Rahmen des demographischen Wandels ist mit einer weiteren deutlichen Zunahme der Prävalenz der Aortenstenose zu rechnen. Bei symptomatischen Patienten mit Aortenstenose besteht grundsätzlich eine Indikation zum Aortenklappenersatz. Die meisten Patienten zeigen nach Klappenersatz eine symptomatische Verbesserung, allerdings verbessert sich ein Teil der Patienten nur in geringem Ausmaß und die Sterblichkeit im ersten Jahr nach Klappeneingriff liegt zwischen 15 und 20 %.

Biopsie-basierte Analysen der Fibrose (Quantifizierung ohne entsprechende Subtypisierung) wurden bei Patienten mit chirurgischem Klappenersatz durchgeführt. Da sich der chirurgische Klappenersatz und die Katheter-basierte Herzklappenimplantation (TAVI) im Hinblick auf Patientenauswahl und Behandlungserfolg unterscheiden, können die Befunde, die im Zusammenhang mit operativen Eingriffen erhoben wurden, jedoch nicht auf TAVI-Patienten übertragen werden (Hein S, Circulation 2003; Krayenbuehl HP, Circulation 1989; Weidemann F, Circulation 2009, Azevedo CF, J Am Coll Cardiol 2010; Mack MJ, New Engl J of Med 2019; Herrmann S, J Am Coll Cardiol 2011).

Eine zunehmende Anzahl von Studien evaluiert die Fibrose über kardiale Magnetresonanztomographie (MRI) (Musa TA, Circulation 2018; Treibel TA, Am Coll Cardiol 2018, Chen H, Clinical Radiology 2018; Lee H, JACC Cardiovascular Imaging 2018; Bing R, JACC Cardiovascular Imaging 2019). Keine dieser Publikationen diskutiert, eine Subpopulation von Aortenstenosepatienten basierend auf der Fibrosequantifizierung zu identifizieren, oder gar diese antifibrotisch zu behandeln.

Spironolacton hemmt den Aldosteronrezeptor, dessen Aktivierung zu einer vermehrten Kollagenbildung und Kollagenquervernetzung führt (Ravassa S, Eur J Heart Fail 2018). Nach Behandlung alter normotensiver Ratten mit Spironolacton wurde eine verringerte Dichte des kardialen Kollagens beobachtet (Lacolley et al., 2001). Eine frühzeitige Behandlung spontan hypertensiver Ratten mit Spironolacton verringerte die Häufigkeit von Myokardinfarkten durch Verbesserung der Myokardfunktion und Verringerung von Fibrose (Cezar et al., 2015). Spironolacton hat die Zulassung für die Hypertoniebehandlung und die Ödembehandlung, nicht primär für die Fibrosebehandlung. Gleichwohl wird die Substanz bei Herzinsuffizienz und nach Myokardinfarkt wegen ihres günstigen Einfluss auf den reversen Remodellingprozess eingesetzt (Ponikowski et al., 2016).

Hydralazin oder Dihydralazin ist zugelassen zur Hypertoniebehandlung, aber nicht zur Fibrosebehandlung. In einer früheren Publikation der vorliegenden Erfinder (Tampe B, EBioMedicine 2015) ergab sich der Befund, dass Hydralazin demethylierende Eigenschaften besitzt und DNA-Methylierung eine profibrotische Stoffwechsellage generiert. So führt die Methylierung u. a. des RASAL1-Gens zur Fibroseprogression. RASAL1 hat hemmende Effekte auf RASGTPasen (und damit stimulierende Effekte auf RAS-Aktivität), und RAS-Aktivierung führt zur Fibroseinduktion. Es gibt auch Belege dafür, dass die Transkriptionsinhibierung von RASAL1 durch Promotormethylierung zum Fortschreiten kardialer Fibrose beiträgt (Xu et al., 2015). Ein potentielle Verwendbarkeit von Hydralazin bei der Behandlung chronischer Herzinsuffizienz und chronischen Nierenversagens wurde von Zeisberg et al. (Zeisberg et al., 2016) diskutiert.

Ziel der vorliegenden Erfindung ist es, den Behandlungserfolg nach TAVI zu verbessern im Sinne einer Verbesserung der Lebensqualität, aber ganz besonders im Sinne einer Reduktion der Sterblichkeit.

**ZUSAMMENFASSUNG DER ERFINDUNG**

In einer prospektiven Analyse bei Patienten mit hochgradiger Aortenklappenstenose haben die vorliegenden Erfinder die Abhängigkeit der Sterblichkeit 1 Jahr nach TAVI von verschiedenen Parametern untersucht. Schließlich erwies sich die myokardiale Fibrose als entscheidender Parameter. Myokardiale Fibrose wurde histologisch zum Zeitpunkt der kathetergestützten Aortenklappenimplantation (TAVI) ermittelt. Die Patienten wurde stratifiziert und in zwei Subgruppen mit Fibroselast oberhalb (MF+) und unterhalb (MF-) des Medians (11 % Fibroseanteil der Querschnittsfläche) unterteilt. MF+ erwies sich in der 1-Jahres-Nachsorge (6-24 Monate, im Mittel 11 Monate) als Multivariat-unabhängiger Prädiktor der kardiovaskulären Sterblichkeit (HR 27; 2,037-369,0; P = 0,013). Univariat war MF+ auch ein Prädiktor der Gesamtsterblichkeit (HR 2,8; 1,2 - 6,6; P = 0,02). Von 100 Patienten verstarben 14 an kardiovaskulären Ursachen, davon 9 Arrhythmie-assoziiert. In der MF- Gruppe betrug die kardiovaskulare Sterblichkeit 2 % und in der MF+ Gruppe 26,5 %. Durch eine weitere Stratifizierung der Fibrose in Fibrosesubtypen kann eine weitere Steigerung der Risikoprädiktion erreicht werden. (Siehe Beispiele hierin sowie Puls et al., 2020). Entsprechend stellt die vorliegende Offenbarung ein Verfahren bereit zum Identifizieren von Patienten mit Aortenstenose, die einer Katheter-basierten Aortenklappenimplantation (transcatheter aortic valve implantation; TAVI) unterzogen werden, die von einer Behandlung mit einem anti-Fibrose-Mittel profitieren würden, wobei das Verfahren die Schritt umfasst:

(a) Bereitstellen einer Myokardbiopsieprobe des Patienten;
(b) Bestimmen des prozentualen Anteils an myokardialer Fibrose bezogen auf die Querschnittsfläche des Myokard; und
(c) Bestimmen ob der in Schritt (b) bestimmte prozentuale Anteil an myokardialer Fibrose gleich oder oberhalb eines klinisch signifikanten Schwellenwertes liegt;

wobei wenn der bestimmte prozentuale Anteil an myokardialer Fibrose bezogen auf die Querschnittsfläche des Myokard gleich des oder größer als der klinisch signifikante Schwellenwert ist, dies indiziert, dass der TAVI Patient von einer Behandlung mit einem anti-Fibrose-Mittel profitieren würde.

Gleichsam stellt die vorliegende Offenbarung ein Verfahren bereit zum Überwachen des Behandlungserfolgs von Patienten mit Aortenstenose, die einer Katheter-basierten Aortenklappenimplantation (transcatheter aortic valve implantation; TAVI) unterzogen wurden, wobei das Verfahren die Schritte umfasst:

(a) Bereitstellen einer Myokardbiopsieprobe des Patienten;
(b) Bestimmen des prozentualen Anteils an myokardialer Fibrose bezogen auf die Querschnittsfläche des Myokard; und
(c) Bestimmen ob der in Schritt (b) bestimmte prozentuale Anteil an myokardialer Fibrose untererhalb eines klinisch signifikanten Schwellenwertes liegt;

wobei eine Stagnation oder eine Reduktion des bestimmten prozentualen Anteils an myokardialer Fibrose bezogen auf die Querschnittsfläche des Myokard von einem Wert gleich oder über dem Schwellenwert auf einen Wert unter den klinisch signifikanten Schwellenwert einen Behandlungserfolg indiziert.

In diesem Zusammenhang wird auch die Verwendung eines Kits für eine Masson-Trichrom-Färbung zur Bestimmung des Biomarkers "myokardiale Fibrose von gleich oder mehr als 11% der Querschnittsfläche des Myokard" als Prädiktor für die kardiovaskuläre Mortalität bei der Behandlung und/oder Prävention von Patienten mit Aortenstenose, die einer Katheter-basierten Aortenklappenimplantation (transcatheter aortic valve implantation; TAVI) unterzogen werden, offenbart.

Ebenfalls offenbart wird die Verwendung eines Kits für eine Masson-Trichrom-Färbung zur Bestimmung des Biomarkers "myokardiale Fibrose von gleich oder mehr als 11% der Querschnittsfläche des Myokard" als Prädiktor für den Behandlungserfolg bei der Nachsorge von Patienten mit Aortenstenose, die einer Katheter-basierten Aortenklappenimplantation (transcatheter aortic valve implantation; TAVI) unterzogen wurden.

Auf Basis dieser Erkenntnis fanden die vorliegenden Erfinder eine adjuvante antifibrotische Therapie zu etablieren mit dem Ziel der Sterblichkeitssenkung in einer durch den aufgefundenen Biomarker identifizierten Subgruppe von Patienten mit Aortenstenose mit hoher kardiovaskulärer Sterblichkeit.

Erfindungsgemäß wird ein Anti-Fibrose-Mittel zur Anwendung in der Behandlung und/oder Prävention von Patienten mit Aortenstenose, die einer Katheter-basierten Aortenklappenimplantation (transcatheter aortic valve implantation; TAVI) unterzogen werden, wobei die Patienten dadurch gekennzeichnet sind, dass sie eine myokardiale Fibrose aufweisen, die gleich oder höher als ein klinisch signifikanter Schwellenwert der Querschnittsfläche ist, bereitgestellt.

Dieses Anti-Fibrose-Mittel umfasst eine Kombination von Spironolacton mit Hydralazin und/oder Dihydralazin.. In einer bevorzugten Ausführungsform wird das Hydralazin oder Dihydralazin in einer Dosis verabreicht wird, die deutlich niedriger ist als die Dosierung von Hydralazin oder Dihydralazin zur Behandlung von Hypertonie.

In diesem Zusammenhang kann die optimale Dosis von Hydralazin oder Dihydralazin in der Anti-Fibrose-Behandlung eines Patienten mit Aortenstenose, der einer Katheter-basierten Aortenklappenimplantation (transcatheter aortic valve implantation; TAVI) unterzogen wird oder wurde, wobei der Patient dadurch gekennzeichnet ist, dass er eine myokardiale Fibrose aufweist, die gleich oder höher als ein klinisch signifikanter Schwellenwert der Querschnittsfläche ist, mit einem Verfahren bestimmt werden, welches folgendes umfasst:

Bestimmen des Acetylierungs-Phänotyps durch Messen des Spiegels an Hydralazin/Dihydralazin und 3-hydroxymethyltriazolophthalazin (HOMTP) im Urin des Patienten; und/oder Genotypisieren von Genen des Patienten, deren Genprodukte in die Metabolisierung von Hydralazin oder Dihydralazin involviert sind, insbesondere durch Genotypisierung des NAT1 und/oder NAT2 Gens.

Weitere vorteilhafte Ausführungen sind nachstehend ausgeführt.

## DETAILLIERTE BESCHREIBUNG

Unsere in den Beispielen aufgeführte Studie ist die erste, die eine Myokardbiopsie als Basis für eine Biomarkersuche für hohe kardiovaskuläre Mortalität ein Jahr nach TAVI evaluiert. Neben zahlreichen anderen Untersuchungen, die in der Biopsie durchgeführt wurden (next generation sequencing, hoch auflösende Mikroskopie, Chromosemen-Instabilität etc.) wurde eine Fibroseanalyse (Quantifizierung mit Subtypisierung der Fibrose) durchgeführt. Die Myokardfibrose, gemessen als prozentualer Fibroseanteil der histologischen Biopsiequerschnittsfläche erwies sich als unabhäniger Prädiktor der kardiovaskulären Mortalität.

Entsprechend betrifft die vorliegende Offenbarung ein Verfahren zum Identifizieren von Patienten mit Aortenstenose, die einer Katheter-basierten Aortenklappenimplantation (transcatheter aortic valve implantation; TAVI) unterzogen werden, die von einer Behandlung mit einem Anti-Fibrose-Mittel profitieren würden, wobei das Verfahren die Schritt umfasst:

(a) Bereitstellen einer Myokardbiopsieprobe des Patienten;
(b) Bestimmen des prozentualen Anteils an myokardialer Fibrose bezogen auf die Querschnittsfläche des Myokard; und
(c) Bestimmen ob der in Schritt (b) bestimmte prozentuale Anteil an myokardialer Fibrose gleich oder oberhalb eines klinisch signifikanten Schwellenwertes liegt;

wobei wenn der bestimmte prozentuale Anteil an myokardialer Fibrose bezogen auf die Querschnittsfläche des Myokard gleich oder größer als des klinisch signifikanten Schwellenwertes ist, dies indiziert, dass der TAVI Patient von einer Behandlung mit einem Anti-Fibrose-Mittel profitieren würde.

Die Aortenstenose (Aortenklappenstenose) ist der am häufigsten vorkommende Herzklappenfehler, und ist gekennzeichnet durch eine Verhärtung und Verengung (Stenose) der Herzklappe am Ausgang der linken Herzkammer. Aufgrund dieser Verengung muss das Herz mehr Kraft aufbringen, um dagegen anzupumpen. Bei symptomatischen Patienten mit Aortenstenose (AS) besteht grundsätzlich eine Indikation zum Aortenklappenersatz. Dieser kann einerseits durch eine Operation am offenen Herzen erfolgen, oder minimalinvasiv mittels Katheter. Der Fachmann bezeichnet dieses Verfahren als TAVI (transcatheter aortic valve implantation, zu Deutsch: Transkatheter-Aortenklappenimplantation). Es gehört somit zum allgemeinen Fachwissen des Fachmanns, einen Patienten zu identifizieren, der eine Aortenstenose aufweist und einer Katheter-basierten Aortenklappenimplantation (transcatheter aortic valve implantation; TAVI) unterzogen werden soll. Jedoch nur das hierin offenbarte Verfahren erlaubt es dem Fachmann, eine Untergruppe dieser Patienten zu identifizieren, welches erlaubt, diese Untergruppe adjuvant mit einem Anti-Fibrose-Mittel zu behandeln, um den Heilungserfolg zu erhöhen und die Mortalität nach TAVI zu reduzieren. Kern dieses Verfahrens ist die Identifikation eines univariaten Prädiktors, nämlich ein klinisch signifikanter Schwellenwert des prozentualen Anteils an myokardialer Fibrose bezogen auf die Querschnittsfläche des Myokards.

Einerseits - und bevorzugt - kann dieser Prädiktor mittels einer Myokardbiopsie bestimmt werden, genauer einer Myokardbiopsie des linken Ventrikels. Verfahren zur Durchführung einer Myokardbiopsie sind dem Fachmann bekannt. Wie in den nachfolgenden Beispielen veranschaulicht, kann dann der prozentuale Anteil an myokardialer Fibrose bezogen auf die Querschnittsfläche des Myokard nach einer histologischen Analyse bestimmt werden, insbesondere nach einer Masson-Trichrom-Färbung der Biopsieprobe.

Die myokardiale Fibrose kann jedoch auch durch kardiale Magnetresonanztomographie (MRT) bestimmt werden (vgl. Ref 12-15). Die MRT hat den großen Vorteil, dass sie nicht invasiv ist und somit problemlos sequentielle Untersuchungen ermöglicht. Die Quantifizierung der irreversiblen Ersatzfibrose durch späte Gadoliniumanreicherung (late gadolinium enhancement; LGE) ist gut validiert und wird durch leistungsfähige prognostische Daten (Ref 16) unterstützt. Die potenziell reversible diffuse interstitielle Fibrose kann jedoch nur mit der T1-Mapping-Technik gemessen werden, die sich in einem frühen Entwicklungsstadium befindet. So gelten die Myokardbiopsie und die histologische Analyse nach wie vor als die Goldstandardbewertungen der Myokardfibrose (Ref 16).

Dennoch könnte man grundsätzlich das hierin offenbarte Verfahren noch verallgemeinern. Es beträfe dann ein Verfahren zum Identifizieren von Patienten mit Aortenstenose, die einer Katheter-basierten Aortenklappenimplantation (transcatheter aortic valve implantation; TAVI) unterzogen werden, die von einer Behandlung mit einem Anti-Fibrose-Mittel profitieren würden, wobei das Verfahren die Schritt umfasst:

(a) Bestimmen des prozentualen Anteils an myokardialer Fibrose bezogen auf eine Querschnittsfläche des Myokard; und

(b) Bestimmen ob der in Schritt (a) bestimmte prozentuale Anteil an myokardialer Fibrose gleich oder oberhalb eines klinisch signifikanten Schwellenwertes liegt;

wobei wenn der bestimmte prozentuale Anteil an myokardialer Fibrose bezogen auf die Querschnittsfläche des Myokard gleich des oder größer als der klinisch signifikante Schwellenwert ist, dies indiziert, dass der TAVI Patient von einer Behandlung mit einem Anti-Fibrose-Mittel profitieren würde.

Der klinisch signifikante Schwellenwert ist in einigen Ausführungsformen ein Wert zwischen 6 % und 85 % ist, bevorzugt ein Wert zwischen 7 % und 70%, stärker bevorzugt ein Wert zwischen 8 % und 60%, stärker bevorzugt ein Wert zwischen 9 % und 50 %, stärker bevorzugt ein Wert zwischen 10 % und 40 %, stärker bevorzugt ein Wert zwischen 10 % und 30%, stärker bevorzugt ein Wert zwischen 11 % und 20 %, wie beispielsweise 11 %, 12 %, 13 %, 14 %, 15 %, 16 %, 17 %, 18 %, 19 %, oder 20 %.

Die Beispiele zeigen, dass durch den Biomarker "myokardiale Fibrose über 11 % der histologischen Querschnittsfläche" eine Subpopulation von Aortenstenosen beschrieben wird mit einer Sterblichkeit von 26,5 % ein Jahr nach dem TAVI Eingriff. Die Mortalität in Abwesenheit dieses Biomarkers (myokardiale Fibrose < 11 %) beträgt 2 % pro Jahr. Entsprechend beträgt der klinisch signifikante Schwellenwert vorzugsweise 11 %.

Eine weitere Steigerung der Risikoprädiktion wird durch zusätzliche Bestimmung der Fibrosesubtypen erreicht. Nach den aktuellen Richtlinien (Ref. 1) werden vier Subtypen von schweren AS mit Hilfe von echokardiographischen Parametern definiert:

1. Normale/erhaltene Auswurffraktion, hoher Gradient AS (NEF-HG AS):

$$\text{LV-EF} \geq 50\%,$$

$$v_{max} \geq 4 \text{ m/s oder } P_{mean} \geq 40 \text{ mmHg},$$

$$\text{AVA} \leq 1{,}0 \text{ cm}^2$$

2. Niedrige/reduzierte Auswurffraktion, hoher Gradient AS (LEF-HG):

$$\text{LV-EF} < 50\%,$$

$$v_{max} \geq 4 \text{ m/s oder } P_{mean} \geq 40 \text{ mmHg},$$

$$\text{AVA} \leq 1{,}0 \text{ cm}^2$$

3. Niedrige/reduzierte Auswurffraktion, niedriger Gradient AS ("klassisch" niedrig-Fluss, niedriger Gradient AS) (LEF-LG AS):

$$\text{LV-EF} < 50\%,$$

$$v_{max} < 4 \text{ m/s und } P_{mittel} < 40 \text{ mmHg},$$

$$\text{AVA} \leq 1{,}0 \text{ cm}^2,$$

$$\text{Schlagvolumenindex (stroke volume index; SVI)} \leq 35 \text{ ml/m}^2$$

4. Paradox niedrigfließend, niedriger Gradient AS (PLF-LG AS):

$$\text{LV-EF} \geq 50\%,$$

vmax <4 m/s und Pmittel <40 mmHg,

AVA ≤1,0 cm² und auf Körperoberfläche indexierte AVA ≤0,6 cm²/ m²,

SVI ≤35 ml/m²

Die einzelnen Parameter und deren Bestimmung sind in den nachfolgenden Beispielen und in Ref. 1 beschrieben.

Folglich umfasst das Verfahren in weiteren Ausführungsformen als zusätzlichen Schritt das Bestimmen des hämodynamischen Subtyps der Aortenstenose, wobei wenn der Patient den PLF-LG AS Subtyp oder den LEF-LG AS Subtyp aufweist dies indiziert, dass der TAVI Patient von einer Behandlung mit einem Anti-Fibrose-Mittel profitieren würde. Insbesondere wenn der Patient den PLF-LG AS Subtyp aufweist indiziert dies, dass der TAVI Patient von einer Behandlung mit einem Anti-Fibrose-Mittel profitieren würde.

Wenn der Patient bereits einer TAVI unterzogen wurde, und vor oder während des Eingriffs keine Myokardbiopsieprobe genommen wurde, so kann das hierin offenbare Verfahren dennoch vorteilhaterweise in der Nachbehandlung angewendet werden. In diesem Falle könnte man eine Myokardbiopsie, bevorzugt des linken Ventrikels, nach dem Eingriff nachholen, um den prozentualen Anteil an myokardialer Fibrose bezogen auf die Querschnittsfläche des Myokards zu bestimmen. Da dieser Parameter ein Prädiktor ist, empfiehlt es sich, die Myokardbiopsie in diesem Falle möglichst zeitnah nach dem Eingriff nachzuholen.

Somit wird hierin auch ein Verfahren bereitgestellt zum Überwachen des Behandlungserfolgs von Patienten mit Aortenstenose, die einer Katheter-basierten Aortenklappenimplantation (transcatheter aortic valve implantation; TAVI) unterzogen wurden, wobei das Verfahren die Schritte umfasst:

(a) Bereitstellen einer Myokardbiopsieprobe des Patienten;
(b) Bestimmen des prozentualen Anteils an myokardialer Fibrose bezogen auf die Querschnittsfläche des Myokard; und
(c) Bestimmen ob der in Schritt (b) bestimmte prozentuale Anteil an myokardialer Fibrose untererhalb eines klinisch signifikanten Schwellenwertes liegt;

wobei eine Stagnation oder eine Reduktion des bestimmten prozentualen Anteils an myokardialer Fibrose bezogen auf die Querschnittsfläche des Myokard von einem Wert gleich oder über dem Schwellenwert auf einen Wert unter den klinisch signifikanten Schwellenwert einen Behandlungserfolg indiziert.

Analog zu dem anderen Verfahren kann Schritt (b) beispielsweise mittels einer histologischen Analyse bestimmt werden, bevorzugt nach Masson-Trichrom-Färbung der Myokardbiopsieprobe. Alternativ oder zusätzlich könnte der Patient mittels MRT untersucht werden, wie vorstehend weiter aufgeführt. Somit könnte auch dieses Verfahren grundsätzlich weiter gefasst werden, nämlich als ein Verfahren zum Überwachen des Behandlungserfolgs von Patienten mit Aortenstenose, die einer Katheter-basierten Aortenklappenimplantation (transcatheter aortic valve implantation; TAVI) unterzogen wurden, wobei das Verfahren die Schritte umfasst:

(a) Bestimmen des prozentualen Anteils an myokardialer Fibrose bezogen auf die Querschnittsfläche des Myokard; und
(b) Bestimmen ob der in Schritt (a) bestimmte prozentuale Anteil an myokardialer Fibrose unterhalb eines klinisch signifikanten Schwellenwertes liegt;

wobei eine Stagnation oder eine Reduktion des bestimmten prozentualen Anteils an myokardialer Fibrose bezogen auf die Querschnittsfläche des Myokard von einem Wert gleich oder über dem Schwellenwert auf einen Wert unter den klinisch signifikanten Schwellenwert einen Behandlungserfolg indiziert.

Wie zuvor ist der klinisch signifikante Schwellenwert in Ausführungsformen ein Wert zwischen 6 % und 85 % ist, bevorzugt ein Wert zwischen 7 % und 70%, stärker bevorzugt ein Wert zwischen 8 % und 60%, stärker bevorzugt ein Wert zwischen 9 % und 50 %, stärker bevorzugt ein Wert zwischen 10 % und 40 %, stärker bevorzugt ein Wert zwischen 10 % und 30%, stärker bevorzugt ein Wert zwischen 11 % und 20 %, wie beispielsweise 11 %, 12 %, 13 %, 14 %, 15 %, 16 %, 17 %, 18 %, 19 %, oder 20 %.. In besonders bevorzugten Ausführungsformen beträgt der klinisch signifikante Schwellenwert 11 %.

In Ausführungsformen können die Schritte des Bestimmens des prozentualen Anteils an myokardialer Fibrose bezogen auf die Querschnittsfläche des Myokard und des Bestimmens ob der bestimmte prozentuale Anteil an myokardialer Fibrose unterhalb eines klinisch signifikanten Schwellenwertes liegt auch maschinell und/oder automatisiert durchgeführt werden. Dies kann besonders vorteilhaft bei einer Bestimmung des prozentualen Anteils an myokardialer Fibrose mittels MRT durchgeführt werden. Somit werden auch die vorstehenden Verfahren in Computer-implementierter Form offenbart, insbesondere ein Datenträger mit einer Software oder einem Algorithmus, der die Durchführung der vorstehend offenbarten Verfahren erlaubt.

Die vorstehend angeführten Verfahren sind nicht nur von Interesse für Patienten, Ärzte und Produzenten von Anti-FibroseMitteln. Auch stellen diese Verfahren eine neue interessante Anwendungsmöglichkeit für Hersteller von Diagnosekits bereit. So betrifft die vorliegende Offenbarung auch

(i) die Verwendung eines Kits für eine Masson-Trichrom-Färbung zur Bestimmung des Biomarkers "myokardiale Fibrose von gleich oder mehr als 11% der Querschnittsfläche des Myokards" als Prädiktor für die kardiovaskuläre Mortalität bei der Behandlung und/oder Prävention von Patienten mit Aortenstenose, die einer Katheter-basierten Aortenklappenimplantation (transcatheter aortic valve implantation; TAVI) unterzogen werden; und

(ii) die Verwendung eines Kits für eine Masson-Trichrom-Färbung zur Bestimmung des Biomarkers "myokardiale Fibrose von gleich oder mehr als 11% der Querschnittsfläche" als Prädiktor für den Behandlungserfolg bei der Nachsorge von Patienten mit Aortenstenose, die einer Katheter-basierten Aortenklappenimplantation (transcatheter aortic valve implantation; TAVI) unterzogen wurden.

Ausgehend von der Entdeckung, dass die Fibrose im Myokard den direkten Grund für die hohe Mortalität darstellt, entwickelten die Erfinder eine Therapie der Fibrose mit dem Ziel der Regression.

Entsprechend betrifft die vorliegende Offenbarung ein Anti-Fibrose-Mittel zur Anwendung in der Behandlung und/oder Prävention von Patienten mit Aortenstenose, die einer Katheter-basierten Aortenklappenimplantation (transcatheter aortic valve implantation; TAVI) unterzogen werden, wobei die Patienten dadurch gekennzeichnet sind, dass sie eine myokardiale Fibrose aufweisen, die gleich oder höher als ein klinisch signifikanter Schwellenwert der Querschnittsfläche ist.

Gleichsam zu den Verfahren betrifft die vorliegende Offenbarung auch Anti-Fibrose-Mittel zur Anwendung in der Nachbehandlung von Patienten mit Aortenstenose, die einer Katheter-basierten Aortenklappenimplantation (transcatheter aortic valve implantation; TAVI) unterzogen wurden, wobei die Patienten dadurch gekennzeichnet sind, dass sie eine myokardiale Fibrose aufweisen, die gleich oder höher als ein klinisch signifikanter Schwellenwert der Querschnittsfläche ist.

Bevorzugt sind die zu behandelnden Patienten durch das vorstehend offenbarte Verfahren identifiziert worden. Entsprechend werden Ausführungsformen ins Auge gefasst, in denen die prozentuale myokardiale Fibrose des Patienten mittels Myokardbiopsie, bevorzugt des linken Ventrikels, und einer histologischen Analyse bestimmt wurde, bevorzugt nach Masson-Trichrom-Färbung. Zusätzlich oder alternativ wurde die prozentuale myokardiale Fibrose des Patienten mittels kardialer Magnetresonanztomographie (MRT) bestimmt, insbesondere mittels Bildgebung durch späte Gadoliniumanreicherung und/oder T1-Mapping. Wie in den Beispielen aufgezeigt ist die Behandlung mit einem Anti-Fibrose-Mittel insbesondere vorteilhaft für Patienten mit einer Aortenstenose des Suptyps PLF-LG AS oder des Subtyps LEF-LG AS. Besonders vorteilhaft ist die Behandlung mit einem Anti-Fibrose-Mittel für einen Patienten mit einer Aortenstenose des Suptyps PLF-LG AS.

In Ausführungsformen ist der klinisch signifikante Schwellenwert ein Wert zwischen 6 % und 85 % ist, bevorzugt ein Wert zwischen 7 % und 70%, stärker bevorzugt ein Wert zwischen 8 % und 60%, stärker bevorzugt ein Wert zwischen 9 % und 50 %, stärker bevorzugt ein Wert zwischen 10 % und 40 %, stärker bevorzugt ein Wert zwischen 10 % und 30%, stärker bevorzugt ein Wert zwischen 11 % und 20 %, wie beispielsweise 11 %, 12 %, 13 %, 14 %, 15 %, 16 %, 17 %, 18 %, 19 %, oder 20 %. In besonders bevorzugten Ausführungsformen beträgt der klinisch signifikante Schwellenwert 11 %.

Das Anti-Fibrose-Mittel der vorliegenden Erfindung kann als eine Kombinationstherapie aus zwei bereits zugelassenen Substanzen (Spironolacton und Hydralazin bzw. Dihydralazin) eingesetzt werden.

Die vorliegende Erfindung betrifft daher ein Anti-Fibrose-Mittel basierend auf (i) Spironolacton und/oder (ii) mindestens eines von Hydralazin und Dihydralazin. In einer bevorzugten Ausführungsform ist das Anti-Fibrose-Mittel eine Kombination von (i) Spironolacton und (ii) mindestens einem von Hydralazin und Dihydralazin, wobei eine Kombinationstherapie mit (i) Spironolacton und (ii) Hydralazin besonders bevorzugt ist.

Spironolacton hemmt den Aldosteronrezeptor, dessen Aktivierung zu einer vermehrten Kollagenbildung und Kollagenquervernetzung führt (Ravassa S, Eur J Heart Fail 2018). Nach Behandlung alter normotensiver Ratten mit Spironolacton wurde eine verringerte Dichte des kardialen Kollagens beobachtet (Lacolley et al., 2001). Eine frühzeitige Behandlung spontan hypertensiver Ratten mit Spironolacton verringerte die Häufigkeit von Myokardinfarkten durch Verbesserung der Myokardfunktion und Verringerung von Fibrose (Cezar et al., 2015). Spironolacton hat bereits eine Zulassung für die Hypertoniebehandlung und die Ödembehandlung. Gleichwohl wird die Substanz bei Herzinsuffizienz und nach Myokardinfarkt auch wegen ihres günstigen Einfluss auf den reversen Remodelingprozess eingesetzt (Ponikowski et al., 2016).

Hydralazin oder Dihydralazin ist zugelassen zur Hypertoniebehandlung, aber nicht zur Fibrosebehandlung. Die plausible Eignung von Hydralazin zur Fibrosebehandlung beruht auf einer Publikation der Erfinder (Tampe B, EBioMedicine 2015). Hierbei ergab sich der Befund, dass Hydralazin demethylierende Eigenschaften besitzt und dadurch der DNA-Methylierung, welche eine profibrotische Stoffwechsellage generiert, entgegenwirkt. So führt die Methylierung u. a. des RASAL1-Gens zur Fibroseprogression. RASAL1 hat hemmende Effekte auf RASGTPasen (und damit stimulierende Effekte auf RAS-Aktivität), und RAS-Aktivierung führt zur Fibroseinduktion. Es gibt auch Belege dafür, dass die Transkriptionsinhibierung von RASAL1 durch Promotormethylierung zum Fortschreiten kardialer Fibrose beiträgt (Xu et al., 2015). Ein potentielle Verwendbarkeit von Hydralazin bei der Behandlung chronischer Herzinsuffizienz und chronischen Nierenversagens wurde von Zeisberg et al. (Zeisberg et al., 2016) diskutiert. Es konnte gezeigt werden, dass eine niedrige Dosis von Hydralazin durch eine Demethylierung die Progression der chronischen Niereninsuffizienz durch Fibrose verzögert.

Entscheidend für diese Wirkung ist, dass eine niedrige Dosierung von Hydralazin eingesetzt wird, deutlich niedriger als die zur Hypertoniebehandlung eingesetzte. Hohe Dosen von Hydralazin zeigen demgegenüber ungünstige Effekte, die den günstigen antifibrotischen Effekten der niedrigen Dosis entgegenwirken. Entsprechend ist bevorzugt, dass das Hydralazin oder Dihydralazin in einer Konzentration verabreicht wird, die niedriger ist als für die Hypertoniebehandlung, insbesondere wobei die Konzentration so niedrig ausgewählt wird, dass sie eine demethylierende Wirkung aufweist. Die Dosierung von Hydralazin oder Dihydralazin zur Behandlung von Hypertonie beträgt mindestens 2 x 25 mg pro Tag (ein Patient, bei dem diese niedrige Dosis wirksam zur Behandlung von Hypertonie ist, für den ist sehr wahrscheinlich, dass er ein langsamer Metabolisierer ist) und bis zu 4 x 100 mg pro Tag. Tatsächlich werden die oben beschriebenen antifibrotischen Effekte jedoch bereits bei 5-50 mg pro Tag erreicht.

Hydralazin wird im Menschen durch Acetylierung metabolisiert. Es existieren genetische Polymorphismen die bewirken, dass Hydralazin schnell oder langsam metabolisiert wird. Die Tatsache, dass Hydralazin einerseits nur in einer niedrigen Konzentration therapeutisch/anti-fibrotisch wirksam ist, und andererseits in der Population unterschiedliche Metabolisierertypen vorhanden sind, die dementsprechend eine therapeutisch wirksame Konzentration unterschiedlich erreichen, hat dazu geführt das bisherige kardiologische Untersuchungen zu Hydralazin widersprüchliche Ergebnisse produzierten und eine Aortenklappenstenose in neueren Publikationen als Kontraindikation für die Verabreichung von Hydralazin aufgeführt wird (Sawhney et al, Am J Geriatr Cardiol. 2003 und Mangla et al, Indian Heart Journal 2016). Entsprechend ist es bevorzugt, dass die Dosierung für den Patienten personalisiert wird. So wird vorteilhafterweise das Hydralazin oder Dihydralazin an schnelle Metabolisierer in einer Dosierung im Bereich von 2 x 15,1 mg pro Tag bis 2 x 25 mg pro Tag verabreicht, bevorzugt in einer Dosierung im Bereich von 2 x 16 mg pro Tag bis 2 x 25 mg pro Tag, stärker bevorzugt in einer Dosierung im Bereich von 2 x 17 mg pro Tag bis 2 x 25 mg pro Tag, stärker bevorzugt in einer Dosierung im Bereich von 2 x 18 mg pro Tag bis 2 x 25 mg pro Tag, stärker bevorzugt in einer Dosierung im Bereich von 2 x 19 mg pro Tag bis 2 x 25 mg pro Tag, stärker bevorzugt in einer Dosierung im Bereich von 2 x 20 mg pro Tag bis 2 x 25 mg pro Tag, stärker bevorzugt in einer Dosierung im Bereich von 2 x 21 mg pro Tag bis 2 x 25 mg pro Tag, stärker bevorzugt in einer Dosierung im Bereich von 2 x 22 mg pro Tag bis 2 x 25 mg pro Tag, stärker bevorzugt in einer Dosierung im Bereich von 2 x 23 mg pro Tag bis 2 x 25 mg pro Tag, stärker bevorzugt in einer Dosierung im Bereich von 2 x 24 mg pro Tag bis 2 x 25 mg pro Tag, bevorzugt in einer Dosierung im Bereich von 2 x 16 mg pro Tag bis 2 x 24 mg pro Tag, stärker bevorzugt in einer Dosierung im Bereich von 2 x 17 mg pro Tag bis 2 x 24 mg pro Tag, stärker bevorzugt in einer Dosierung im Bereich von 2 x 18 mg pro Tag bis 2 x 24 mg pro Tag, stärker bevorzugt in einer Dosierung im Bereich von 2 x 19 mg pro Tag bis 2 x 24 mg pro Tag, stärker bevorzugt in einer Dosierung im Bereich von 2 x 20 mg pro Tag bis 2 x 24 mg pro Tag, stärker bevorzugt in einer Dosierung im Bereich von 2 x 21 mg pro Tag bis 2 x 24 mg pro Tag, stärker bevorzugt in einer Dosierung im Bereich von 2 x 22 mg pro Tag bis 2 x 24 mg pro Tag, stärker bevorzugt in einer Dosierung im Bereich von 2 x 23 mg pro Tag bis 2 x 24 mg pro Tag, insbesondere bevorzugte Dosierungen für schnelle Metabolisierer sind 2 x 20 mg pro Tag, 2 x 21 mg pro Tag, 2 x 22 mg pro Tag, 2 x 23 mg pro Tag, 2 x 24 mg pro Tag und 2 x 25 mg pro Tag.

Vorteilhafterweise wird das Hydralazin oder Dihydralazin an langsame Metabolisierer in einer Dosierung im Bereich von 2 x 5 bis 2 x 15 mg pro Tag verabreicht, bevorzugt in einer Dosierung im Bereich von 2 x 6 mg pro Tag bis 2 x 15 mg pro Tag, stärker bevorzugt in einer Dosierung im Bereich von 2 x 7 mg pro Tag bis 2 x 15 mg pro Tag, stärker bevorzugt in einer Dosierung im Bereich von 2 x 8 mg pro Tag bis 2 x 15 mg pro Tag, stärker bevorzugt in einer Dosierung im Bereich von 2 x 9 mg pro Tag bis 2 x 15 mg pro Tag, stärker bevorzugt in einer Dosierung im Bereich von 2 x 10 mg pro Tag bis 2 x 15 mg pro Tag, stärker bevorzugt in einer Dosierung im Bereich von 2 x 11 mg pro Tag bis 2 x 15 mg pro Tag, stärker bevorzugt in einer Dosierung im Bereich von 2 x 12 mg pro Tag bis 2 x 15 mg pro Tag, stärker bevorzugt in einer Dosierung im Bereich von 2 x 12 mg pro Tag bis 2 x 14 mg pro Tag, stärker bevorzugt in einer Dosierung im Bereich von 2 x 12 mg pro Tag bis 2 x 13 mg pro Tag, insbesondere bevorzugte Dosierungen für schnelle Metabolisierer sind 2 x 5 mg pro Tag, 2 x 10 mg pro Tag, 2 x 11 mg pro Tag, 2 x 12 mg pro Tag, 2 x 13 mg pro Tag, 2 x 14 mg pro Tag und 2 x 15 mg pro Tag; besonders bevorzugt beträgt die Dosierung für langsame Metabolisierer 2 x 12,5 mg pro Tag.

Zusätzlich offenbart wird auch die Identifikation des Acetylierungstyps der zu behandelnden Patienten. Genauer wird ein Verfahren bereitgestellt zum Bestimmen der optimalen Dosis von Hydralazin oder Dihydralazin in der Anti-Fibrose-Behandlung eines Patienten mit Aortenstenose, der einer Katheter-basierten Aortenklappenimplantation (transcatheter aortic valve implantation; TAVI) unterzogen wird oder wurde, wobei der Patient dadurch gekennzeichnet ist, dass er eine myokardiale Fibrose aufweist, die gleich oder höher als ein klinisch signifikanter Schwellenwert der Querschnittsfläche ist; wobei das Verfahren umfasst:

(a) Bestimmen des Acetylierungs-Phänotyps durch Messen des Spiegels an Hydralazin/Dihydralazin und 3-hydroxymethyltriazolophthalazin (HOMTP) im Urin des Patienten; und/oder
(b) Genotypisieren von Genen des Patienten, deren Genprodukte in die Metabolisierung von Hydralazin oder Dihydralazin involviert sind, insbesondere durch Genotypisierung des NAT1 und/oder NAT2 Gens.

Der Patient ist derselbe wie durch die Verfahren identifiziert und wie für das Behandlungsverfahren beschrieben. Entsprechend ist in Ausführungsformen der klinisch signifikante Schwellenwert ein Wert zwischen 6 % und 85 % ist, bevorzugt ein Wert zwischen 7 % und 70%, stärker bevorzugt ein Wert zwischen 8 % und 60%, stärker bevorzugt ein Wert zwischen 9 % und 50 %, stärker bevorzugt ein Wert zwischen 10 % und 40 %, stärker bevorzugt ein Wert zwischen 10 % und 30%, stärker bevorzugt ein Wert zwischen 11 % und 20 %, wie beispielsweise 11 %, 12 %, 13 %, 14 %, 15 %, 16 %, 17 %, 18 %, 19 %, oder 20 %. Bevorzugt beträgt der klinisch signifikante Schwellenwert 11 %. In Ausführungsformen wird die prozentuale myokardiale Fibrose mittels Myokardbiopsie und einer histologischen Analyse bestimmt, bevorzugt nach Masson-Trichrom-Färbung. Bevorzugt ist die Myokardbiopsie eine Myokardbiopsie des linken Ventrikels. Alternativ oder zusätzlich dazu kann die prozentuale myokardiale Fibrose mittels kardialer Magnetresonanztomographie (MRT) bestimmt werden, insbesondere mittels Bildgebung durch späte Gadoliniumanreicherung und/oder T1-Mapping. In Ausführungsformen weist der Patient eine Aortenstenose des Suptyps PLF-LG AS oder des Subtyps LEF-LG AS auf. In besonderen Ausführungsformen handelt es sich um einen Patienten mit einer Aortenstenose des Suptyps PLF-LG AS.

Dem Fachmann sind mehrere Möglichkeiten bekannt, zu bestimmen, ob jemand ein schneller oder langsamer Metabolisierer von Hydralazin/Dihydralazin ist. Beispielsweise lässt sich die Differenzierung mittels des Verfahrens beschrieben in Timbrell et al. 1980 (Ref. 25) bestimmen. In Kürze: Unter Verwendung von Gaschromatographie und/oder Hochdruck-Flüssigchromatographie werden zum Zeitpunkt 0 und nach 24 h die Menge an Hydralazin und dessen Metabolisierungsprodukt 3-hydroxymethyltriazolophthalazin (HOMPT) bestimmt. Das Stoffwechselverhältnis HOMTP:Hydralazin beträgt für langsame Metabolisierer (Acetylierer) etwa 1,6 und für schnelle Metabolisierer (Acetylierer) etwa 14,9. Weitere Details zu der Methode können Timbrell et al. 1980 (Ref. 25) entnommen werden. Dem Fachmann sind jedoch auch andere Alternativen bekannt. So beschreibt Garcés-Eisele et al. 2014 (Ref. 26) die Bestimmung des Phänotyps durch das Bestimmen von pharmakokinetischen Parametern von Hydralazin/Dihydralazin im Serum des Patienten.

Neben dem Phänotyp lässt sich die Fähigkeit zum Verstoffwechseln (Acetylieren) von Hydralazin oder Dihydralazin auch mittels Genotypisierung, also auf der Genebene, bestimmen. Genetische Varianten der Gene NAT1 und NAT2 wurden in der Vergangenheit bereits mit ihrer Wirkung auf den Stoffwechsel von Wirkstoffen in Verbindung gebracht. Sowohl NAT 1 als auch NAT 2 sind polymorphe Gene, für NAT1 sind 28 Allele bekannt und für NAT2 sind 88 allelische Varianten bekannt. Per Definition werden NAT1*4 und NAT2*4 als Referenzen (Wildtyp) angesehen, unter Befolgung der Konsensus-Richtlinien und Nomenklatur des Arylamine N-acetyltransferase Gene Nomenclature Committee. Die weiteren Varianten unterscheiden sich von diesen durch einen oder mehreren SNP (small nucleotide polymorphism).

Diverse Allele von NAT1 resultieren in einem Phänotyp, der dem der Referenz NAT1 *4 gleicht (*20, *21, *23, *24, *25, *27; schnelle Acetylierer/Metabolisierer), einige verleihen einen langsamen Acetylierungsphänotyp (*14A, *14B, *17, *22) oder führen zu abgeschnittenen Proteinen ohne enzymatische Aktivität (*15, *19A, *19B).

| NAT1 Allel | Nucleotidaustausche und rs Identifier(s) | Aminosäureaustausche | Phänotyp |
|---|---|---|---|
| NAT1*4 | Referenz | Referenz | Referenz/ Schnell |
| NAT1*3 | 1095C>A (rs15561) | | |
| NAT1*5 | 350-351GG>CC (rs72554606)<br>497-499 GGG>CCC (rs72554608)<br>884A>G (rs55793712)<br>Δ976 (rs72554612)<br>Δ1105 (rs72554613) | R117T R166T; E167Q | |
| NAT1*10 | 1088T>A (rs1057126)<br>1095C>A (rs15561) | | |
| NAT1*11A | -344C>T (rs4986988)<br>-40A>T (rs4986989)<br>445G>A (rs4987076) | V1491<br>T153T (still) S214A | |

(fortgesetzt)

| NAT1 Allel | Nucleotidaustausche und rs Identifier(s) | Aminosäureaustausche | Phänotyp |
|---|---|---|---|
| | 459G>A (rs4986990)<br>640T>G (rs4986783)<br>Δ9 between 1065-1090<br>1095C>A (rs15561) | | |
| NAT1*11B | -344C>T (rs4986988)<br>-40A>T (rs4986989)<br>445G>A (rs4987076)<br>459G>A (rs4986990)<br>640T>G (rs4986783)<br>Δ9 between 1065-1090 | V1491<br>T153T (still) S214A | |
| NAT1*11C | -344C>T (rs4986988)<br>-40A>T (rs4986989)<br>459G>A (rs4986990)<br>640T>G (rs4986783)<br>Δ9 between 1065-1090<br>1095C>A (rs15561) | T153T (still)<br>S214A | |
| NAT1*14A | 560G>A (rs4986782)<br>1088T>A (rs1057126)<br>1095C>A (rs15561) | R187Q | Langsam |
| NAT1*14B | 560G>A (rs4986782) | R187Q | Langsam |
| NAT1*15 | 559C>T (rs5030839) | R187Stop | Inaktiv/Langsam |
| NAT1*16 | [AAA] immediately after 1091 1095C>A (rs15561) | | |
| NAT1*17 | 190C>T (rs56379106) | R64W | Langsam |
| NAT1*18A | Δ3 between 1065-1087 (rs4646271)<br>1088T>A (rs1057126)<br>1095C>A (rs15561) | | |
| NAT1*18B | Δ3 between 1065-1087 (rs4646271) | | |
| NAT1*19A | 97C>T (rs56318881) | | Inaktiv/Langsam |
| NAT1*19B | 97C>T (rs56318881)<br>190C>T (rs56379106) | | Inaktiv/Langsam |
| NAT1*20 | 402T>C (rs146727732) | | wie NAT1*4 |
| NAT1*21 | 613A>G (rs72554609) | | wie NAT1*4 |
| NAT1*22 | 752A>T (rs56172717) | | Langsam |
| NAT1*23 | 777T>C (rs4986991) | | wie NAT1*4 |
| NAT1*24 | 781G>A (rs72554610) | | wie NAT1*4 |
| NAT1*25 | 787A>G (rs72554611) | 1263V | wie NAT1*4 |
| NAT1*26A | [TAA] insertion between 1065 and 1090 1095C>A (rs15561) | | |
| NAT1*26B | [TAA] insertion between 1065 and 1090 | | |
| NAT1*27 | 21T>G (rs4986992)<br>777T>C (rs4986991) | L7L (still) S259S (still) | wie NAT1*4 |
| NAT1*28 | [TAATAA] deletion between 1065 - 1090 | | |
| NAT1*29 | 1088T>A (rs1057126) | | |
| | 1095C>A (rs15561) Δ1025 | | |

11

(fortgesetzt)

| NAT1 Allel | Nucleotidaustausche und rs Identifier(s) | Aminosäureaustausche | Phänotyp |
|---|---|---|---|
| NAT1*30 | 445G>A (rs4987076) | V1491 | |

Für NAT2 wurden 'schnelle' Allele berichtet als NAT2*4, NAT*11A, *12A-C, *13A und *18. NAT2 Allele mit langsamen Acetylierungs-Phänotyp sind *5, *6, *7, *14A und *14B. Weitere Informationen zu NAT2-Allelen, deren Phänotyp und Bestimmung finden sich in Ruiz et al. 2012 (Ref. 26) und Garces-Eisele et al. 2014 (Ref. 27).

| NAT2 Allel | Nucleotidaustausche und rs Identifier(s) | Aminosäureaustausche | Phänotyp |
|---|---|---|---|
| NAT2*4 | Referenz | Referenz | Referenz/ Schnell |
| NAT2*5A | 341T>C (rs1801280)<br>481C>T (rs1799929) | 1114T<br>L161L (still) | Langsam |
| NAT2*5B | 341T>C (rs1801280)<br>481C>T (rs1799929)<br>803A>G (rs1208) | 1114T<br>L161L (still)<br>K268R | Langsam |
| NAT2*5C | 341T>C (rs1801280)<br>803A>G (rs1208) | 1114T<br>K268R | Langsam |
| NAT2*5D | 341T>C (rs1801280) | 1114T | Langsam |
| NAT2*5E | 341T>C (rs1801280)<br>590G>A (rs1799930) | 1114T<br>R197Q | Langsam |
| NAT2*5F | 341T>C (rs1801280)<br>481C>T (rs1799929)<br>759C>T (rs56011192)<br>803A>G (rs1208) | 1114T<br>L161L (still)<br>V253V (still)<br>K268R | Langsam |
| NAT2*5G | 282C>T (rs1041983)<br>341T>C (rs1801280)<br>481C>T (rs1799929)<br>803A>G (rs1208) | Y94Y (still)<br>1114T<br>L161L (still)<br>K268R | Langsam |
| NAT2*5H | 341T>C (rs1801280)<br>481C>T (rs1799929)<br>803A>G (rs1208)<br>Δ859 | 1114T<br>L161L (still)<br>K268R<br>S287 Frameshift | Langsam |
| NAT2*51 | 341T>C (rs1801280)<br>411A>T (rs4986997)<br>481C>T (rs1799929)<br>803A>G (rs1208) | 1114T<br>L137F<br>L161L (still)<br>K268R | Langsam |
| NAT2*5Jᵉ | 282C>T (rs1041983)<br>341T>C (rs1801280)<br>590G>A (rs1799930) | Y94Y (still)<br>1114T<br>R197Q | Langsam |
| NAT2*5K | 282C>T (rs1041983)<br>341T>C (rs1801280) | Y94Y (still)<br>1114T | |
| NAT2*5KAᶠ | 282C>T (rs1041983)<br>341T>C (rs1801280)<br>857G>A (rs1799931) | Y94Y (still)<br>1114T<br>G286E | |
| NAT2*5L | 70T>A (rs45477599)<br>341T>C (rs1801280)<br>481C>T (rs1799929) | L241<br>1114T<br>L161L (still) | |

(fortgesetzt)

| NAT2 Allel | Nucleotidaustausche und rs Identifier(s) | Aminosäureaustausche | Phänotyp |
|---|---|---|---|
| | 803A>G (rs1208) | K268R | |
| NAT2*5M | 341T>C (rs1801280)<br>481C>T (rs1799929)<br>803A>G (rs1208)<br>838G>A (rs56393504) | 1114T<br>L161L (still)<br>K268R<br>V280M | |
| NAT2*5N | 341T>C (rs1801280)<br>472A>C (rs139351995)<br>481C>T (rs1799929)<br>803A>G (rs1208) | 1114T<br>1158L<br>L161L (still)<br>K268R | |
| NAT2*5O | 203G>A (rs72466458)<br>341T>C (rs1801280)<br>481C>T (rs1799929)<br>803A>G (rs1208) | C68Y<br>1114T<br>L161L (still)<br>K268R | |
| NAT2*5P | 282C>T (rs1041983)<br>341T>C (rs1801280)<br>481C>T (rs1799929)<br>578C>T (rs79050330)<br>590G>A (rs1799930)<br>803A>G (rs1208) | Y94Y (still)<br>1114T<br>L161L (still)<br>T193M<br>R197Q<br>K268R | |
| NAT2*5Q[f] | 341T>C (rs1801280)<br>590G>A (rs1799930)<br>803A>G (rs1208) | 1114T<br>R197Q<br>K268R | |
| NAT2*5R[f] | 282C>T (rs1041983)<br>341T>C (rs1801280)<br>590G>A (rs1799930)<br>803A>G (rs1208) | Y94Y (still)<br>1114T<br>R197Q<br>K268R | |
| NAT2*5S[f] | 341T>C (rs1801280)<br>857G>A (rs1799931) | 1114T<br>G286E | |
| NAT2*5T[f] | 282C>T (rs1041983)<br>341T>C (rs1801280)<br>803A>G (rs1208) | Y94Y (still)<br>1114T<br>K268R | |
| NAT2*5TA[f] | 282C>T (rs1041983)<br>341T>C (rs1801280)<br>803A>G (rs1208)<br>857G>A (rs1799931) | Y94Y (still)<br>1114T<br>K268R<br>G286E | |
| NAT2*5U[f] | 282C>T (rs1041983)<br>341T>C (rs1801280)<br>481C>T (rs1799929)<br>590G>A (rs1799930)<br>803A>G (rs1208) | Y94Y (still)<br>1114T<br>L161L (still)<br>R197Q<br>K268R | |
| NAT2*5V[f] | 282C>T (rs1041983)<br>341T>C (rs1801280)<br>481C>T (rs1799929) | Y94Y (still)<br>1114T<br>L161L (still) | |
| NAT2*5VA[f] | 282C>T (rs1041983)<br>341T>C (rs1801280)<br>481C>T (rs1799929) | Y94Y (still)<br>1114T<br>L161L (still) | |

(fortgesetzt)

| NAT2 Allel | Nucleotidaustausche und rs Identifier(s) | Aminosäureaustausche | Phänotyp |
|---|---|---|---|
| | 857G>A (rs1799931) | G286E | |
| NAT2*5W[f] | 341T>C (rs1801280)<br>354T>C (rs146405047)<br>481C>T (rs1799929)<br>803A>G (rs1208) | 1114T<br>N118N (still)<br>L161L (still)<br>K268R | |
| NAT2*5X[f] | 341T>C (rs1801280)<br>403C>G (rs12720065)<br>481C>T (rs1799929)<br>803A>G (rs1208) | 1114T<br>L135V<br>L161L (still)<br>K268R | |
| NAT2*5Y[f] | 341T>C (rs1801280)<br>481C>T (rs1799929)<br>622T>C (rs56387565)<br>803A>G (rs1208) | 1114T<br>L161L (still)<br>Y208H<br>K268R | |
| NAT2*5Z[f] | 191G>A (rs1801279)<br>341T>C (rs1801280)<br>481C>T (rs1799929) | R64Q<br>1114T<br>L161L (still) | |
| NAT2*6A | 282C>T (rs1041983)<br>590G>A (rs1799930) | Y94Y (still)<br>R197Q | Langsam |
| NAT2*6B | 590G>A (rs1799930) | R197Q | Langsam |
| NAT2*6C | 282C>T (rs1041983)<br>590G>A (rs1799930)<br>803A>G (rs1208) | Y94Y (still)<br>R197Q<br>K268R | Langsam |
| NAT2*6D | 111T>C (rs72554615)<br>282C>T (rs1041983)<br>590G>A (rs1799930) | F37F (still)<br>Y94Y (still)<br>R197Q | Langsam |
| NAT2*6E | 481C>T (rs1799929)<br>590G>A (rs1799930) | L161L (still)<br>R197Q | Langsam |
| NAT2*6F | 590G>A (rs1799930)<br>803A>G (rs1208) | R197Q<br>K268R | |
| NAT2*6G | 282C>T (rs1041983)<br>518A>G<br>590G>A (rs1799930) | Y94Y (still)<br>K173R<br>R197Q | |
| NAT2*6H | 282C>T (rs1041983)<br>590G>A (rs1799930)<br>766A>G (rs55700793) | Y94Y (still)<br>R197Q<br>K256E | |
| NAT2*61 | 282C>T (rs1041983)<br>590G>A (rs1799930)<br>838G>A (rs56393504)<br>857G>A (rs1799931) | Y94Y (still)<br>R197Q<br>V280M<br>G286E | |
| NAT2*6J | 282C>T (rs1041983)<br>590G>A (rs1799930)<br>857G>A (rs1799931) | Y94Y (still)<br>R197Q<br>G286E | |
| NAT2*6K | 282C>T (rs1041983)<br>590G>A (rs1799930)<br>638C>T (rs138707146) | Y94Y (still)<br>R197Q<br>P213L | |

(fortgesetzt)

| NAT2 Allel | Nucleotidaustausche und rs Identifier(s) | Aminosäureaustausche | Phänotyp |
|---|---|---|---|
| NAT2*6L | 282C>T (rs1041983)<br>345C>T (rs45532639)<br>590G>A (rs1799930) | Y94Y (still)<br>D115D(still)<br>R197Q | |
| NAT2*6M | 152G>T (rs72466457)<br>282C>T (rs1041983)<br>590G>A (rs1799930) | G51V<br>Y94Y (still)<br>R197Q | |
| NAT2*6N | 282C>T (rs1041983)<br>481C>T (rs1799929)<br>590G>A (rs1799930) | Y94Y (still)<br>L161L (still)<br>R197Q | |
| NAT2*6O<sup>f</sup> | 282C>T (rs1041983)<br>590G>A (rs1799930)<br>838G>A (rs56393504) | Y94Y (still)<br>R197Q<br>V280M | |
| NAT2*6P<sup>f</sup> | 403C>G (rs12720065)<br>590G>A (rs1799930) | L135V<br>R197Q | |
| NAT2*6Q<sup>f</sup> | 282C>T (rs1041983)<br>308C>T<br>590G>A (rs1799930) | Y94Y (still)<br>T1031<br>R197Q | |
| NAT2*6R<sup>f</sup> | 282C>T (rs1041983)<br>481C>T (rs1799929)<br>590G>A (rs1799930)<br>803A>G (rs1208) | Y94Y (still)<br>L161L (still)<br>R197Q<br>K268R | |
| NAT2*6S<sup>f</sup> | 590G>A (rs1799930)<br>857G>A (rs1799931) | R197Q<br>G286E | |
| NAT2*6T<sup>f</sup> | 481C>T (rs1799929)<br>590G>A (rs1799930)<br>857G>A (rs1799931) | L161L (still)<br>R197Q<br>G286E | |
| NAT2*6U<sup>f</sup> | 282C>T (rs1041983)<br>590G>A (rs1799930)<br>579G>T (rs144176822) | Y94Y (still)<br>R197Q<br>T193T (still) | |
| NAT2*6V<sup>f</sup> | 403C>G (rs12720065)<br>590G>A (rs1799930)<br>838G>A (rs56393504) | L135V<br>R197Q<br>V280M | |
| NAT2*7A | 857G>A (rs1799931) | G286E | Langsam; Substrat abhängig? |
| NAT2*7B | 282C>T (rs1041983)<br>857G>A (rs1799931) | Y94Y (still)<br>G286E | Langsam; Substrat abhängig? |
| NAT2*7C | 282C>T (rs1041983)<br>803A>G (rs1208)<br>857G>A (rs1799931) | Y94Y (still)<br>K268R<br>G286E | |
| NAT2*7D | 191G>A (rs1801279)<br>282C>T (rs1041983)<br>857G>A (rs1799931) | R64Q<br>Y94Y (still)<br>G286E | |
| NAT2*7E<sup>f</sup> | 282C>T (rs1041983)<br>481C>T (rs1799929)<br>857G>A (rs1799931) | Y94Y (still)<br>L161L (still)<br>G286E | |
| NAT2*7F<sup>f</sup> | 282C>T (rs1041983) | Y94Y (still) | |

(fortgesetzt)

| NAT2 Allel | Nucleotidaustausche und rs Identifier(s) | Aminosäureaustausche | Phänotyp |
|---|---|---|---|
| | 481C>T (rs1799929)<br>803A>G (rs1208)<br>857G>A (rs1799931) | L161L (still)<br>K268R<br>G286E | |
| NAT2*7G[f] | 226T>G<br>282C>T (rs1041983)<br>857G>A (rs1799931) | Y76D<br>Y94Y (still)<br>G286E | |
| NAT2*10 | 499G>A (rs72554617) | E167K | Langsam; Substrat abhängig? |
| NAT2*11A | 481C>T (rs1799929) | L161L (still) | Schnell |
| NAT2*11B | 481C>T (rs1799929)<br>$\Delta^{859}$ | L161L (still)<br>S287 Frameshift | |
| NAT2*12A | 803A>G (rs1208) | K268R | Schnell |
| NAT2*12B | 282C>T (rs1041983)<br>803A>G (rs1208) | Y94Y (still)<br>K268R | Schnell |
| NAT2*12C | 481C>T (rs1799929)<br>803A>G (rs1208) | L161L (still)<br>K268R | Schnell |
| NAT2*12D | 364G>A (rs4986996)<br>803A>G (rs1208) | D122N<br>K268R | Langsam |
| NAT2*12E | 282C>T (rs1041983)<br>578C>T (rs79050330)<br>803A>G (rs1208) | Y94Y (still)<br>T193M<br>K268R | |
| NAT2*12F | 622T>C (rs56387565)<br>803A>G (rs1208) | Y208H<br>K268R | |
| NAT2*12G | 609G>T (rs45618543)<br>803A>G (rs1208) | E203D<br>K268R | |
| NAT2*12H | 403C>G (rs12720065)<br>803A>G (rs1208) | L135V<br>K268R | |
| NAT2*121 | 228C>T (rs72466459)<br>803A>G (rs1208) | Y76Y (still)<br>K268R | |
| NAT2*12J | 29T>C (rs72466456)<br>803A>G (rs1208) | 110T<br>K268R | |
| NAT2*12K[f] | 472A>C (rs139351995)<br>803A>G (rs1208) | 1158L<br>K268R | |
| NAT2*12L[f] | 665T>G<br>803A>G (rs1208) | F222C<br>K268R | |
| NAT2*12M[f] | 282C>T (rs1041983)<br>481C>T (rs1799929)<br>803A>G (rs1208) | Y94Y (still)<br>L161L (still)<br>K268R | |
| NAT2*12N | 121A>T (rs149283608)<br>803A>G (rs1208) | N41Y<br>K268R | |
| NAT2*12O | 29T>C (rs72466456)<br>609G>T (rs45618543)<br>803A>G (rs1208) | 110T<br>E203D<br>K268R | |
| NAT2*12P[f] | 472A>C (rs139351995)<br>481C>T (rs1799929) | 1158L<br>L161L (still) | |

(fortgesetzt)

| NAT2 Allel | Nucleotidaustausche und rs Identifier(s) | Aminosäureaustausche | Phänotyp |
|---|---|---|---|
| | 803A>G (rs1208) | K268R | |
| NAT2*13A | 282C>T (rs1041983) | Y94Y (still) | Schnell |
| NAT2*13B | 282C>T (rs1041983)<br>578C>T (rs79050330) | Y94Y (still)<br>T193M | |
| NAT2*13Cᶠ | 282C>T (rs1041983)<br>665T>G | Y94Y (still)<br>F222C | |
| NAT2*13D | 282C>T (rs1041983)<br>766A>G (rs55700793) | Y94Y (still)<br>K256E | |
| NAT2*13E | 282C>T (rs1041983)<br>641C>T | Y94Y (still)<br>T2141 | |
| NAT2*13F | 282C>T (rs1041983)<br>838G>A (rs56393504) | Y94Y (still)<br>V280M | |
| NAT2*13G | 282C>T (rs1041983)<br>472A>C (rs139351995) | Y94Y (still)<br>1158L | |
| NAT2*14A | 191G>A (rs1801279) | R64Q | Langsam |
| NAT2*14B | 191G>A (rs1801279)<br>282C>T (rs1041983) | R64Q<br>Y94Y (still) | Langsam |
| NAT2*14C | 191G>A (rs1801279)<br>341T>C (rs1801280)<br>481C>T (rs1799929)<br>803A>G (rs1208) | R64Q<br>1114T<br>L161L (still)<br>K268R | Langsam |
| NAT2*14D | 191G>A (rs1801279)<br>282C>T (rs1041983)<br>590G>A (rs1799930) | R64Q<br>Y94Y (still)<br>R197Q | Langsam |
| NAT2*14E | 191G>A (rs1801279)<br>803A>G (rs1208) | R64Q<br>K268R | Langsam |
| NAT2*14F | 191G>A (rs1801279)<br>341T>C (rs1801280)<br>803A>G (rs1208) | R64Q<br>1114T<br>K268R | Langsam |
| NAT2*14G | 191G>A (rs1801279)<br>282C>T (rs1041983)<br>803A>G (rs1208) | R64Q<br>Y94Y (still)<br>K268R | Langsam |
| NAT2*14H | 191G>A (rs1801279)<br>282C>T (rs1041983)<br>683C>T (rs45518335) | R64Q<br>Y94Y (still)<br>P228L | |
| NAT2*141 | 191G>A (rs1801279)<br>481C>T (rs1799929)<br>803A>G (rs1208) | R64Q<br>L161L (still)<br>K268R | |
| NAT2*14Jᶠ | 191G>A (rs1801279)<br>282C>T (rs1041983)<br>633G>A | R64Q<br>Y94Y (still)<br>T211T (still) | |
| NAT2*14K | 191G>A (rs1801279)<br>282C>T (rs1041983)<br>838G>A (rs56393504) | R64Q<br>Y94Y (still)<br>V280M | |

(fortgesetzt)

| NAT2 Allel | Nucleotidaustausche und rs Identifier(s) | Aminosäureaustausche | Phänotyp |
|---|---|---|---|
| NAT2*14L | 7A>G (rs200893121) 191G>A (rs1801279) 282C>T (rs1041983) | 13V R64Q Y94Y (still) | |
| NAT2*17 | 434A>C (rs72554616) | Q145P | Langsam |
| NAT2*18 | 845A>C (rs56054745) | K282T | Schnell |
| NAT2*19 | 190C>T (rs1805158) | R64W | Langsam |
| NAT2*20 | 600A>G (rs72466461) | E200E (still) | |
| NAT2*21 | 458C>T (rs72466460) | T1531 | |
| NAT2*22 | 609G>T (rs45618543) | E203D | |
| NAT2*23 | 70T>A (rs45477599) | L241 | |
| NAT2*24 | 403C>G (rs12720065) | L135V | |
| NAT2*25 | 665T>G | F222C | |
| NAT2*26 | 809T>C | 1270T | |
| NAT2*27 | 589C>T | R197Stop | |

Die vorliegende Offenbarung wird ergänzt und veranschaulicht durch die nachfolgenden Beispiele und die angehängten Abbildungen.

**BESCHREIBUNG DER ABBILDUNGEN**

**Abbildung 1: Ausmaß der Myokardfibrose bei verschiedenen hämodynamischen Subtypen schwerer AS** (Mittelwert und Standardabweichung angegeben). Die Fibrose wurde in Masson Trichrome gefärbten (MTS) Biopsieabschnitten als blaue Fläche im Verhältnis zur gesamten Gewebefläche bewertet. NEF-HG: normale EF, hoher Gradient; LEF-HG: reduzierte EF, hoher Gradient; LEF-LG: reduzierte EF, niedriger Gradient (klassische low-flow, low-gradient AS); PLF-LG: paradoxe low-flow, low-gradient AS.

**Abbildung 2: MTC-gefärbte endomyokardiale Biopsien von 4 Patienten mit verschiedenen AS-Subtypen**

**A:** 73 Jahre alter männlicher Patient mit **NEF-HG AS,** KHK angiographisch ausgeschlossen, kein Diabetes, EF vor TAVI 60%, LVEDV 91 ml, LVMI 124 g/m$^2$; geringe MF-Last (5%), überwiegend interstitielle (einschließlich perivaskuläre) Lokalisation; komplikationsloser Langzeitverlauf.
**B:** 67 Jahre alter männlicher Patient mit **LEF-HG AS,** KHK angiographisch ausgeschlossen, kein Diabetes, EF vor TAVI 18%, LVEDV 175 ml, LVMI 224 g/m$^2$; hohe Fibrose-Last (42%) mit subendokardialer und interstitieller Lokalisation; ereignislose Nachbeobachtung mit sehr guter klinischer und echokardiographischer Erholung (EF nach 6 Monaten 52%).
**C:** 89-jährige Patientin mit **LEF-LG AS,** KHK mit chron. RIVA-Verschluss, kein Diabetes, EF vor TAVI 17%, LVEDV 222 ml, LVMI 235 ml/m$^2$; hohe Fibrose-Last (40%), vorwiegend subendokardiale Lokalisation mit massiver Fibroblasteninfiltration des Endokards und der subendokardialen Schicht; fokale Ersatzfibrose; direkter postinterventioneller Verlauf ereignislos, aber Patientin starb 5 Tage nach TAVI an nichtterminierbarer ventrikulärer Tachykardie und erfolgloser Reanimation.
**D:** 76 Jahre alter männlicher Patient mit **PLF-LG AS,** KHK ohne vorherigen Infarkt, Diabetes, EF vor TAVI 54%, LVEDV 82 ml, LVMI 96 g/m$^2$; hohe Fibrose-Last (45%), vorwiegend subendokardial und in geringerem Maße interstitiell lokalisiert; fokale Ersatzfibrose; Patient profitierte klinisch nicht von der TAVI und starb am Tag 125 nach einem kardiochirurgischen Zweit-Eingriff zur Korrektur einer schweren Trikuspidalinsuffizienz.

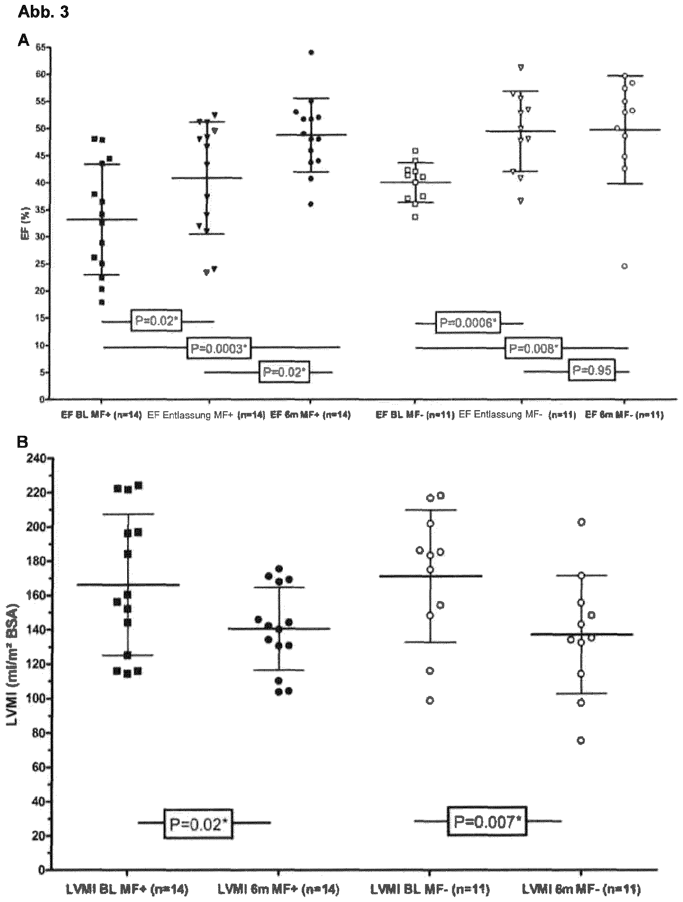

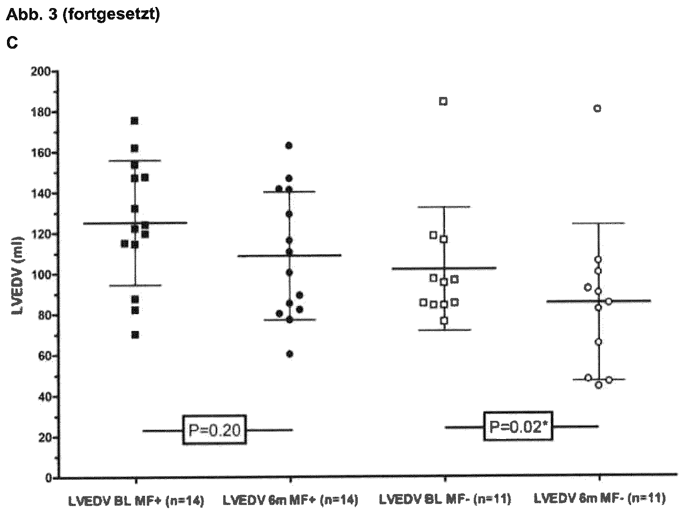

**Abbildung 3: Reverses LV-Remodelling nach 6 Monaten bei Patienten mit reduzierter Ausgangs EF (LEF-HG und LEF-LG AS) in Abhängigkeit von der Fibrose-Last;** nur Patienten mit vorhandenem Follow-up enthalten; A Entwicklung der **EF,** B Entwicklung des linksventrikulären end-diastolischen Voluemens **(LVEDV), C** Entwicklung des linksventrikulären Massenindex **(LVMI);** BL: Anfangswert (Baseline); EF: Auswurffraktion (Ejection Fraction); LVEDV: linksventrikuläres enddiastolisches Volumen; LVMI: linksventrikulärer Massenindex; MF+: myokardiale Fibrose ≥11% (=Median für Gesamtkohorte), MF-: Myokardfibrose <11%.

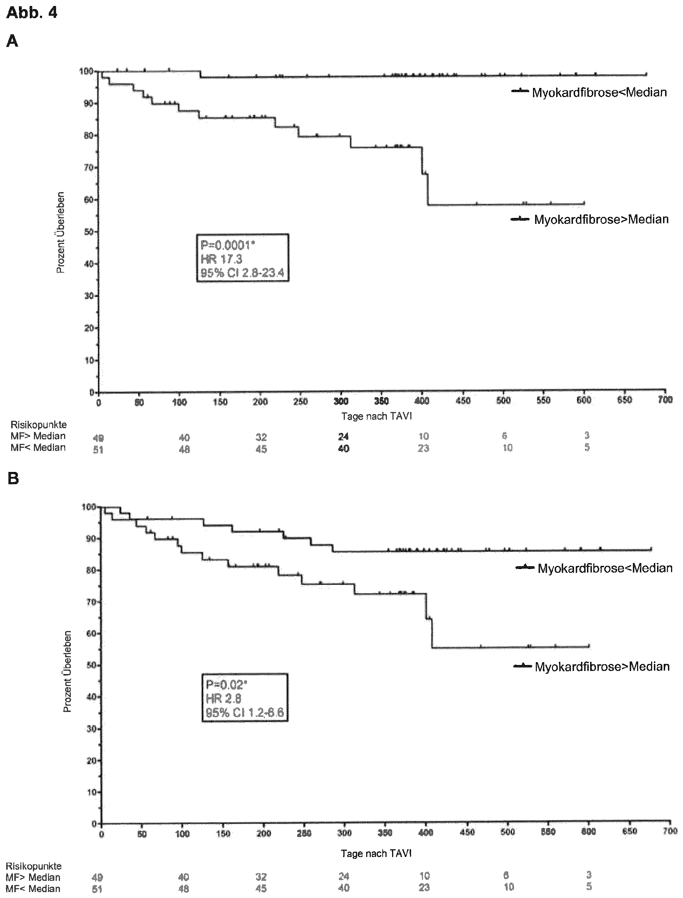

**Abbildung 4: Kardiovaskuläre Mortalität und Gesamtmortalität in Abhängigkeit von der Fibrose-Last;** Kaplan-Meier-Kurven mit Darstellung der kardiovaskulären (A) und der Gesamtmortalität (B) bei Patienten mit Myokardfibrose unterhalb (schwarz) und oberhalb (rot) des Medians.

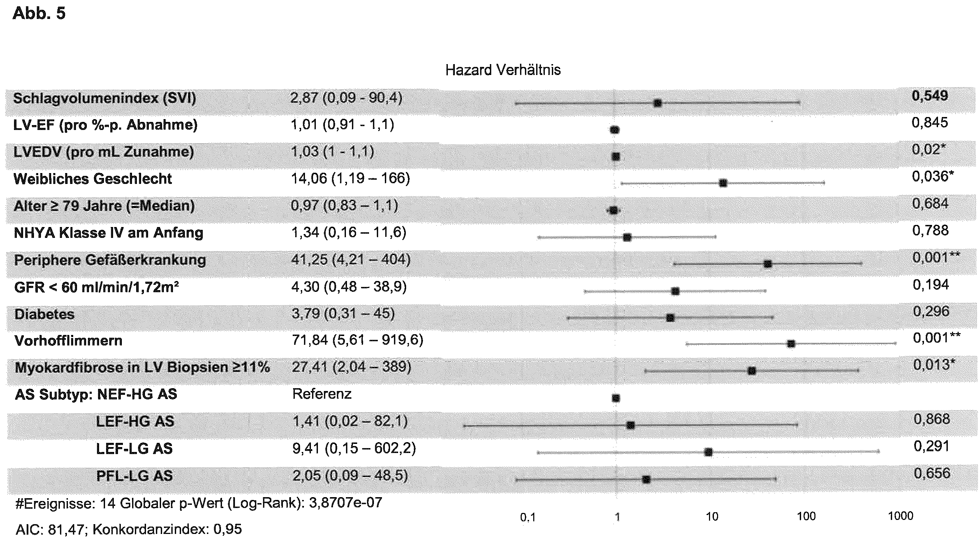

**Abbildung 5: Forest plot: Prädiktoren für die kardiovaskuläre Mortalität während der Nachbeobachtung nach TAVI;** NEF-HG: normale EF, hoher Gradient; LEF-HG: reduzierte EF, hoher Gradient; LEF-LG: reduzierte EF, niedriger Gradient (klassischer Lowflow, niedriger Gradient); PLF-LG: paradoxer Lowflow, niedriger Gradient.

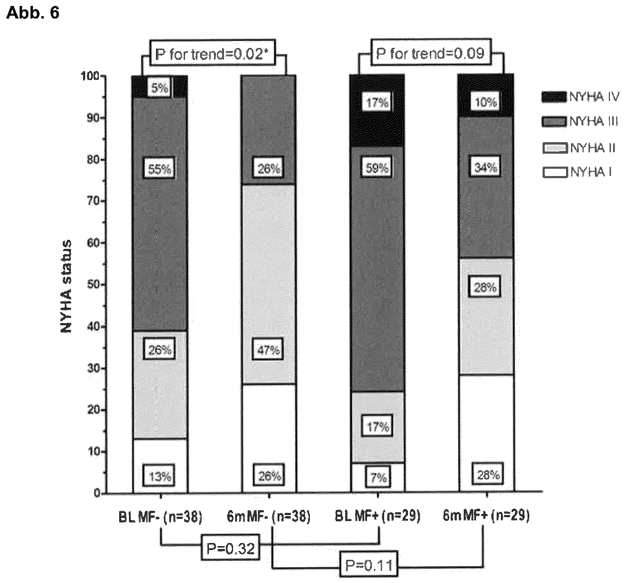

**Abbildung 6: Entwicklung klinischer Herzinsuffizienz-Parameter 6 Monatenach TAVI;** in der gesamten Kohorte konnten signifikante Verbesserungen sowohl bei MF+- als auch bei MF- Patienten in Bezug auf die zurückgelegte Gehstrecke im 6min-Gehtest (6mwt) beobachtet werden (MF+: 193±117 vs. 252±137 m, P=0,006; MF-: 254±115 vs. 254±115). Die Verbesserung der NYHA-Klasse war nur bei MF-Patienten signifikant (MF-: P für Trend 0,02; MF+: P für Trend 0,09).

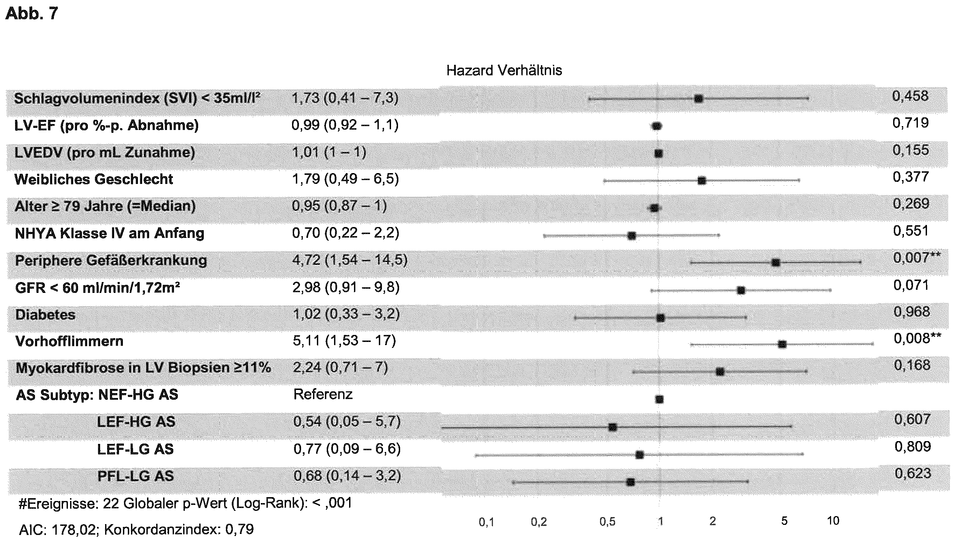

**Abbildung 7: Prädiktoren für die Gesamtmortalität während der Nachbeobachtung;** gezeigt sind Kaplan-Meier-Kurven für die Gesamtmortalität bei Patienten mit Myokardfibrose unterhalb und oberhalb des Medians. In der multivariaten Analyse erwiesen sich nur die periphere arterielle Verschlusskrankheit und Vorhofflimmern als unabhängige Prädiktoren für die Gesamtmortalität.

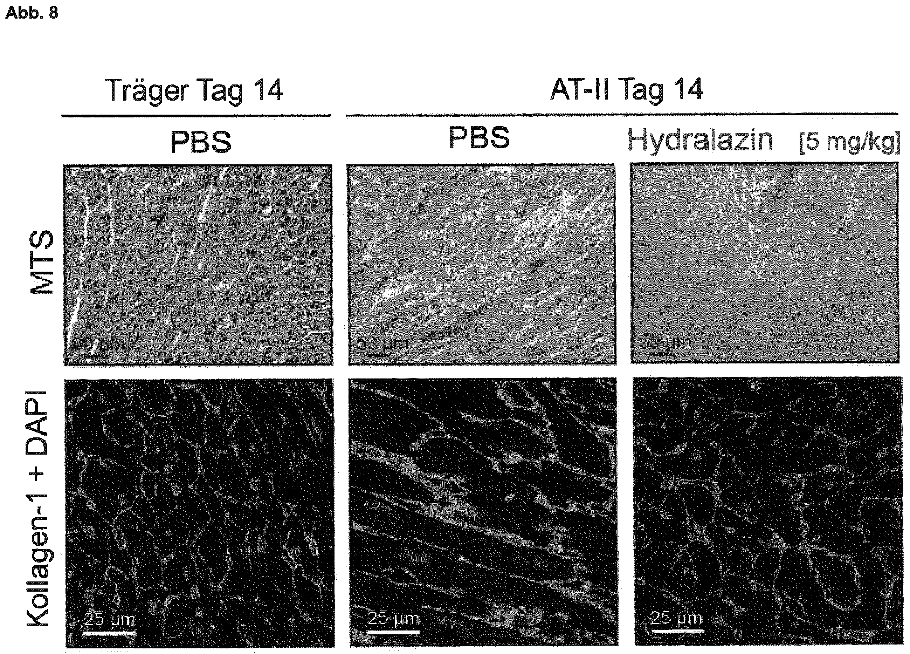

**Abbildung 8: Fibrosereduktion in Mausmodell für Angiotensin II-(AT-II-)induzierte kardiomyozytäre Hypertrophie und kardiale Fibrose durch Behandlung mit geringer Dosis an Hydralazin,** gezeigt sind repräsentative Fluoreszenzbilder einer Kollagen-1 Antikörperfärbung (+DAPI) und einer MTS-Färbung von Herzgewebe aus Mäusen nach Induktion kardiomyozytärer Hypertrophie und kardialer Fibrose mittels AT-II und Behandlung mit 5 mg/kg Hydralazine bzw. PBS (Kontrolle). Behandlung mit Hydralazin reduzierte die durch AT-II induzierte Vergrößerung der Kollagen-1 Bereiche.

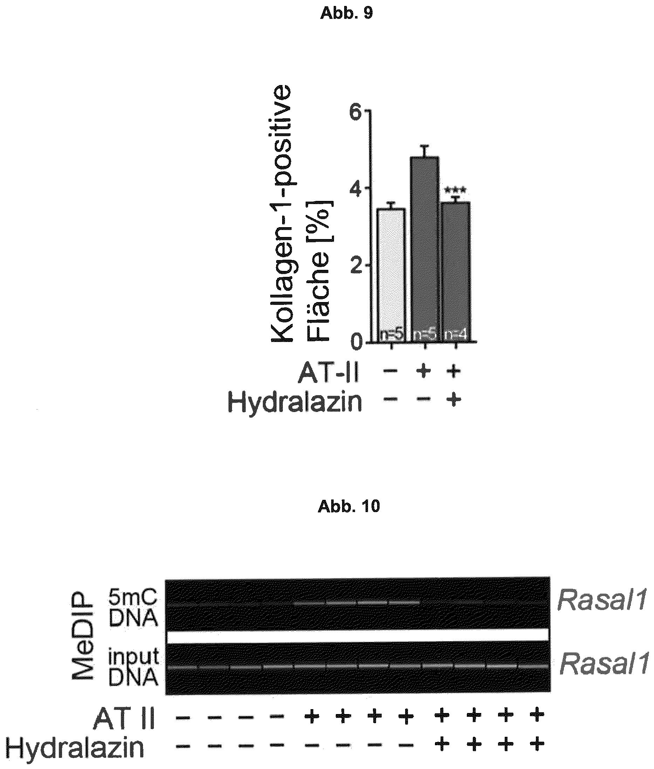

**Abbildung 9: Fibrosereduktion in Mausmodell für Angiotensin II-(AT-II-)induzierte kardiomyozytäre Hypertrophie und kardiale Fibrose durch Behandlung mit geringer Dosis an Hydralazin,** gezeigt sind die Mittelwerte ± Standardabweichung der anhand von Fluoreszenzbildern berechneten prozentualen linksventrikulären interstitiellen Fläche Kollagen-1-positiver Bereiche im Herzgewebe von Mäusen nach Induktion kardiomyozytärer Hypertrophie und kardialer Fibrose mittels AT-II und Behandlung mit 5 mg/kg Hydralazine bzw. PBS (Kontrolle).

**[0062]** * p<0,05, ** p<0,01, *** p<0,001. Die Behandlung mit Hydralazin reduzierte die durch AT-II induzierte Vergrößerung der Kollagen-1-positiven Bereiche deutlich.

**Abbildung 10: Reduktion der RASAL1-Methylierung in Mausmodell für Angiotensin II-(AT-II-)induzierte kardiomyozytäre Hypertrophie und kardiale Fibrose durch Behandlung mit geringer Dosis an Hydralazin,** gezeigt sind die Ergebnisse quantitativer RT-PCT (qRT-PCR) zur Bestimmung von RASAL1 in methylierter DNA (,5mC DNA'; isoliert mittels Methylated DNA Immunoprecipitation, MeDIP) bzw. Input-DNA (ohne MeDIP pull-down) von Mäusen nach Induktion kardiomyozytärer Hypertrophie und kardialer Fibrose mittels AT-II und Behandlung mit 5 mg/kg Hydralazine bzw. PBS (Kontrolle). Die Behandlung mit Hydralazin reduzierte die durch AT-II induzierte Methylierung von RASAL1 deutlich.

## BEISPIELE

### Beispiel 1

Zwischen 1/2017 und 10/2018 haben die Erfinder prospektiv 100 konsekutive Patienten in unsere Studie aufgenommen, die für eine transfemorale TAVI vorgesehen waren. Die Indikation für TAVI basierte auf dem Konsens des Herzteams gemäß aktueller Leitlinien (Ref 1). Die transfemorale TAVI wurde mit Standardtechniken durchgeführt. In den allermeisten Fällen wurde die Sapien-3-Klappe (Edwards Lifesciences Inc., Irvine, CA, USA) implantiert. Zu Beginn wurden transthorakale und transösophageale Echokardiographie (TTE und TEE), ein 6-Minuten-Gehtest (6 minute walking test; 6mwt), Minnesota Living with Heart failure Quality of Life Fragebogen (MLHFQ), NYHA-Status und NT-proBNP-Werte erfasst. Strukturierte Nachsorge-Besuche nach 6 Monaten, 1 Jahr und 2 Jahren umfassten TTE, 6mwt, MLHFQ und NT-proBNP. Die kardiovaskuläre Mortalität während des Follow-up (VARC-2-Definition) (vgl. Ref 6) wurde als primärer klinischer Endpunkt definiert, die Gesamtmortalität als sekundärer Endpunkt. Die lokale Ethikkommission genehmigte die Studie, und eine schriftliche Einwilligung wurde von allen Patienten eingeholt.

### Echokardiographie

Alle Echokardiogramme wurden entweder mit einem Philips ie33- oder einem Philips Epiq7-System durchgeführt, routinemäßig in einem Bildarchivierungs- und Kommunikationssystem aufgezeichnet und von einem einzelnen Untersucher mit Hilfe einer Q Station 3.8.5 (Philips Healthcare) retrospektiv neu ausgewertet. Echokardiographische Messungen wurden wie gemäß gültiger Leitlinien durchgeführt (Ref. 7).

**Echokardiographische Auswertung:**

Die Auswurffraktion (ejection fraction; EF) wurde durch die Scheibchensummationsmethode nach Simpson ermittelt, und die LV-Masse wurde nach der von ASE empfohlenen Würfelformel berechnet (Ref. 7). Die Berechnung der relativen Wanddicke (relative wall thickness; RWT) mit der Formel $(2 \times$ Hinterwanddicke)/LVEDD erlaubte die Kategorisierung der linksventrikulären Hypertrophie entweder als konzentrische (RWT >0,42) oder exzentrische (RWT ≤0,42) Hypertrophie, sowie die Identifizierung eines konzentrischen Remodellings (normale LV-Masse mit erhöhter RWT) (Ref. 7). Der von der Speckle-Tracking-Echokardiographie abgeleitete global longitudinal strain (GLS, endokardiale Deformation) wurde in den drei apikalen Standardansichten gemessen und gemittelt. GLS-Messungen wurden offline vom gleichen Untersucher mit in allen Fällen gleicher Software durchgeführt (Philips Q Station 3.8.5). Das Schlagvolumen wurde mit einem pulsed-wave Doppler im linksventrikulärenAusflusstrakt gemessen und auf die Körperoberfläche (SVI) indiziert. Auf der Grundlage aktueller Leitlinien (Ref. 1) wurden vier Subtypen von schweren AS definiert:

**1. Normale/erhaltene Auswurffraktion, hoherGradient AS (NEF-HG AS):**

$$\text{LV-EF} \geq 50\%,$$

$$\text{vmax} \geq 4 \text{ m/s oder Pmean} \geq 40 \text{ mmHg},$$

$$\text{AVA} \leq 1,0 \text{ cm}^2$$

**2. Niedrige/reduzierte Auswurffraktion, hoher Gradient AS (LEF-HG):**

$$\text{LV-EF} < 50\%,$$

$$\text{vmax} \geq 4 \text{ m/s oder Pmean} \geq 40 \text{ mmHg},$$

$$\text{AVA} \leq 1,0 \text{ cm}^2$$

**3. Niedrige/reduzierte Auswurffraktion, niedriger Gradient AS ("klassisch" low-flow, low-gradient AS) (LEF-LG AS):**

$$\text{LV-EF} < 50\%,$$

$$\text{vmax} < 4 \text{ m/s und Pmittel} < 40 \text{ mmHg},$$

$$\text{AVA} \leq 1,0 \text{ cm}^2,$$

$$\text{Schlagvolumenindex (stroke volume index; SVI)} \leq 35 \text{ ml/m}^2$$

**4. Paradoxe low-flow, low-gradient AS (PLF-LG AS):**

$$\text{LV-EF} \geq 50\%,$$

$$\text{vmax} < 4 \text{ m/s und Pmittel} < 40 \text{ mmHg},$$

# AVA ≤1,0 cm² und indexierte AVA ≤0,6 cm²/ m²,

# SVI ≤35 ml/m²

**Beurteilung der Myokardfibrose in endomyokardialen Biopsien:**

Nach dem Freisetzen der Transkatheter-Klappe wurden noch während der TAVI-Prozedur LV-Biopsien mit einer Biopsiezange (Proflex-Bioptom 7 F, Medical Imaging Systems) aus dem basalen Anteroseptum entnommen, in 10% PFA fixiert und in Paraffin eingebettet. MF wurde mittels quantitativer Morphometrie (Olympus Software cell-Sens 1.6) in Unkenntnis der klinischen und echokardiographischen Befunde bestimmt als blaue Fläche in Masson Trichrome gefärbten (MTS) Biopsieabschnitten (Abschnitt mit positiver Färbung für Kollagen) im Verhältnis zur gesamten Gewebefläche. Die Verteilungsmuster der Myokardfibrose wurde durch Flächenquantifizierung der vordefinierten Einheiten subendokardiale Fibrose, interstitielle Fibrose und Ersatzfibrose (vgl. Ref. 8,9) im Verhältnis zur Gesamtfläche der Fibrose beurteilt.

**Statistische Analyse:**

Die statistische Analyse wurde mittels Graph Pad Prisma Version 4.0 und Statistical Computing Software R (Version 2.15.1; http://www.r-project.org) durchgeführt. Kontinuierliche Variablen werden als Mittelwert ± Standardabweichung dargestellt und wurden mittels t-Test oder Mann-Whitney U-Test für den 2-Gruppen-Vergleich verglichen. Kategorische Variablen werden als absolute Zahlen und Prozentsätze dargestellt und wurden durch Fishers exact test für den 2-Gruppen-Vergleich und durch Pearsons Chi-Square-Test für den Mehrgruppen-Vergleich verglichen. Ein Wert von P<0,05 wurde als statistisch signifikant angesehen.

Die Überlebenszeitanalyse nach TAVI wurde für Mortalität und kardiovaskuläre Mortalität unter Verwendung des R-Paket 'Survival' durchgeführt, durch Kaplan-Meier-Kurven visualisiert wurde, und mittels Log-Rank-Test verglichen.

Als multivariates Modell wurde das Cox Proportional Hazards Model verwendet. Es wurden Überlebenszeitanalysen für bekannte Ergebnisprädiktoren nach Literatur oder früheren Veröffentlichungen unserer Institution durchgeführt (basisdemographische Charakteristika, echokardiographische Messungen, AS-Subtypen, Myokardfibrose). Nur Risikofaktoren, die in sich in univariaten Analysen als signifikant erwiesen, wurden in multivariate Analysen einbezogen.

**Ergebnisse**

Von den 100 Studienteilnehmern litten 40 an NEF-HG AS, 14 an LEF-HG AS, 26 an LEF-LG AS und 16 an PLF-LG AS. Vier Patienten wurden retrospektiv als "mittelschwere bis schwere AS" (MAS) klassifiziert und von Subtypanalysen ausgeschlossen.

**Demographische Basiseigenschaften**

Die basisdemographsichen Charakteristika sind in Tabelle 1 dargestellt. Die Gesamtkohorte (35 Frauen, 65 Männer) war durch ein hohes Alter (Mittelwert 78±7 Jahre) und eine hohe Prävalenz der koronaren Herzkrankheit (KHK, 70%), Vorhofflimmern (43%), Diabetes (45%) und chronische Niereninsuffizienz (chronic kidney disease; CKD, 54%) gekennzeichnet. 74% der Patienten gehörten den NYHA-Klassen III und IV an. Unter allen hämodynamischen Untergruppen stellten die Patienten mit niedrigem Gradient und reduzierter EF (LEF-LG) die kränkste Kohorte mit der höchsten KHK-Prävalenz dar (früherer Myokardinfarkt in 46%, frühere Bypassoperation in 54%). Dabei konnten die Erfinder für 54% der LEF-LG-Patienten nachweisen, dass eine reduzierte LV-EF der Diagnose einer schweren AS vorausging, während eine bereits bestehende systolische Dysfunktion nur bei 21% der Patienten mit reduzierter EF und hohem Gradienten (LEF-HG) dokumentiert war. In der Kohorte mit niedrigem Gradienten und erhaltener EF (PLFLG) waren der Frauenanteil (56%) und die Prävalenz von Vorhofflimmern (69%) unter allen Untergruppen am höchsten.

Um die Ergebnisse der Studie auf die tägliche klinische Praxis anwendbar zu machen, haben die Erfinder TAVI Patienten rekrutiert, ohne KHK oder andere Komorbiditäten auszuschließen (20% aller TAVI-Patienten, die während der Rekrutierungsphase an unserer Institution behandelt wurden). Die KHK-Prävalenz von 70% in unserer Kohorte deutet darauf hin, dass histologische Veränderungen zumindest teilweise auf die Kombination von Aortenstenose und KHK zurückzuführen sind. Unsere Studienkohorte stellt jedoch ein typisches TAVI-Kollektiv dar, weshalb unsere histologischen Ergebnisse auf TAVI-Patienten in der täglichen klinischen Praxis anwendbar sein sollten.

## Quantitative Herzinsuffizienz-Parameter

6mwt Entfernung und NT-proBNP-Spiegel unterschieden sich signifikant zwischen den AS-Subtypen und identifizierten LEF-HG-Patienten konsequent als diejenigen mit der am weitesten fortgeschrittenen und NEF-HG-Patienten als solche mit der am wenigsten weit fortgeschrittenen klinischen Herzinsuffizienz (Tabelle 1). PLF-LG-Patienten zeigten die niedrigsten NT-proBNP-Werte, aber auch die niedrigsten Gehstrecken im 6min-Gehtest aller Subtypen.

## Echokardiographisches linksventrikuläres Remodelling

Echokardiographische Ausgangsmessungen sind in der Tabelle 3 dargestellt. NEF-HG-Patienten zeigten ein normales linksventrikuläres end-diastolisches Volumen bezogen auf die Körperoberfläche (LVEDVi $40\pm15$ ml/m$^2$ BSA), einen deutlich erhöhten linksventrikulären Massenindex (LVMI, $146\pm38$ g/m$^2$ BSA) und eine sehr hohe relative Wanddicke (RWT, $0,67\pm0,13$), was auf eine signifikante konzentrische LV-Hypertrophie hinweist. Im Gegensatz dazu war die LV-Geometrie von LEF-HG-Patienten durch die Kombination von LV-Dilatation und Hypertrophie (exzentrische Hypertrophie) gekennzeichnet, mit dem höchsten LVEDVi ($64\pm20$ ml/m$^2$ BSA), dem höchsten LVMI ($181\pm41$ g/m$^2$ BSA) und der niedrigsten RWT ($0,50\pm0,10$) aller Untergruppen. Diese Progression des LV-Remodelling in Richtung exzentrischer Hypertrophie deutet darauf hin, dass die LEF-HG AS ein fortgeschritteneres Stadium der NEF-HG AS darstellt. Die echokardiographische LV-Morphologie der LEF-LG-Patienten unterschied sich trotz signifikant niedrigerer transaortaler Gradienten nicht signifikant von den LEF-HG-Patienten (Pmean $24\pm7$ vs. $45\pm11$ mmHg, P<0,001). PLF-LG-Patienten hatten das kleinste linksventrikuläre Kavum (LVEDVi $34\pm14$ ml/m$^2$ BSA) und den niedrigsten LVMI unter allen Untergruppen ($128\pm31$ g/m$^2$ BSA). Die relative Wanddicke ($0,64\pm0,07$) und die auf enddiastolische Volumen ($4,04\pm1,32$ g/ml) bezogene LV-Masse waren jedoch genauso hoch wie bei NEF-HG AS, was auf ein signifikantes (maladaptives) konzentrisches Remodelling/Hypertrophie hinweist. Trotz normaler EF zeigte die longitudinale Myokardfunktion eine subklinische systolische LV-Dysfunktion an (GLS $-16\pm2$%).

## Zusammenhang zwischen Myokardfibrose (MF) in endomyokardialen Biopsien und basisdemographischen Charakteristika

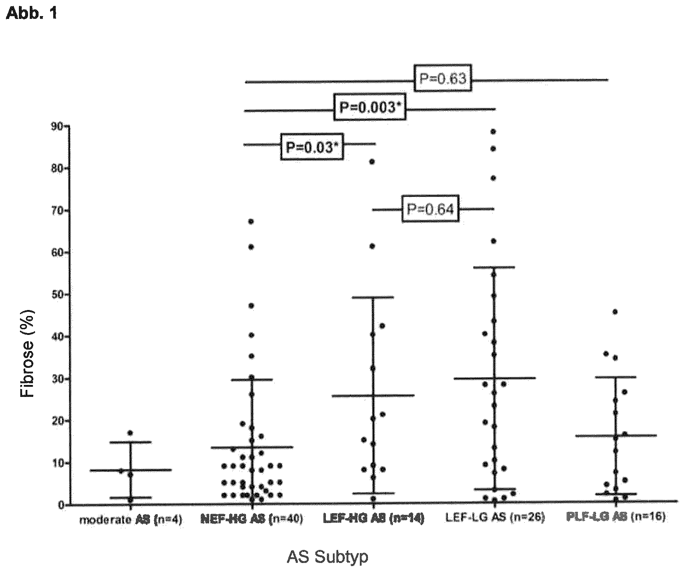

Das Ausmaß der Myokardfibroseunterschied sich signifikant unter den vier hämodynamischen AS-Subtypen (Abb. 1). LEF-HG ($25,6\pm23,3$%) und LEF-LG-Patienten ($29,5\pm26,4$%) zeigten eine signifikant höhere Fibroselast als NEF-HG-Patienten ($13,5\pm16$%; P=0,03 vs. LEF-HG; P=0,003 vs. LEF-LG). Die Fibrose war in PLF-LG ($15,6\pm14,0$) numerisch höher als in NEF-HG AS und niedriger als in LEF-HG und LEF-LG AS, aber beides war nicht signifikant.

Für nachfolgende Analysen unterteilten die Erfinder die Studienteilnehmer entsprechend ihrer Fibroselast in Patienten mit Myokardfibrose über (MF+) und unter (MF-) dem Median der Gesamtkohorte (11%) (Tabelle 2). MF+-Patienten waren signifikant jünger und hatten eine höhere Wahrscheinlichkeit, an Diabetes zu leiden (61% vs. 27%; P=0,0001). Darüber hinaus zeigten sie eine deutlich geringere Gehstrecke im 6mwt ($179\pm122$ vs. $245\pm112$ m, P=0,01). Unter ihnen waren nur 25% aller NYHA-I-Patienten, aber 64% aller NYHA-IV-Patienten. Was echokardiographische Messungen betrifft, so waren MF+-Patienten durch eine signifikant niedrigere EF ($44\pm17$ vs. $55\pm11$%, P=0,0002), eine signifikant schlechtere systolische longitudinale Myokardfunktion (GLS $-13,1\pm4,7$ vs. $-16,3\pm4,2$%, P=0.0001), signifikant größere linke Ventrikel (LVEDV $107\pm45$ vs. $79\pm33$ ml/m$^2$ BSA; P=0,0006), eine signifikant höhere linksventrikuläre Masse (LVMI; $161\pm43$ vs. $142\pm39$ g/m$^2$ BSA, P=0,03) und damit eine signifikant höhere Prävalenz der exzentrischen Hypertrophie (24% vs. 4%, P=0,004) gekennzeichnet. Zusammenfassend lässt sich sagen, dass MF+-Patienten ein höheres Ausmaß an pathologischem LV-Remodelling und klinischer Herzinsuffizienz aufwiesen.

## Verteilungsmuster der Myokardfibrose

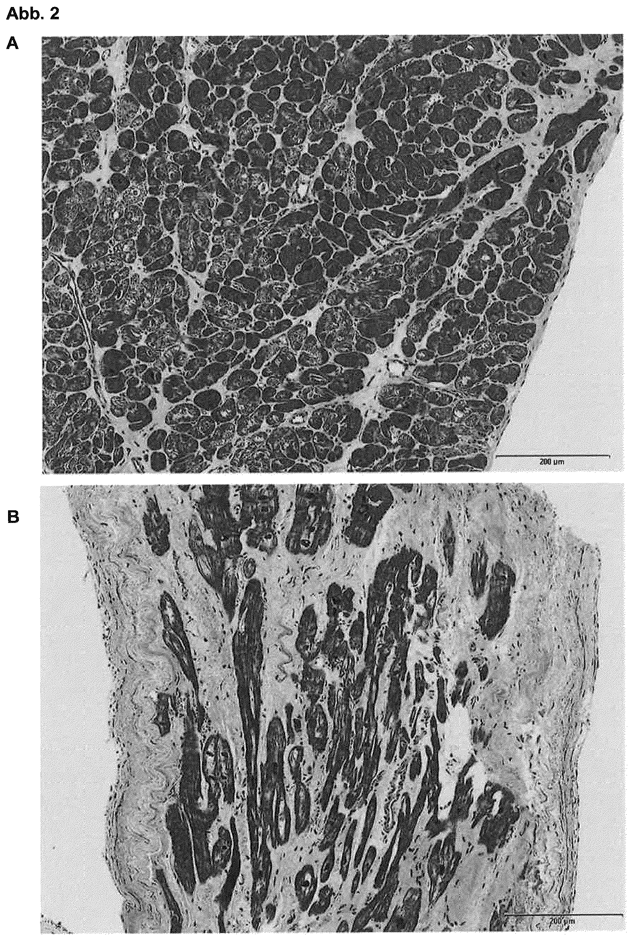

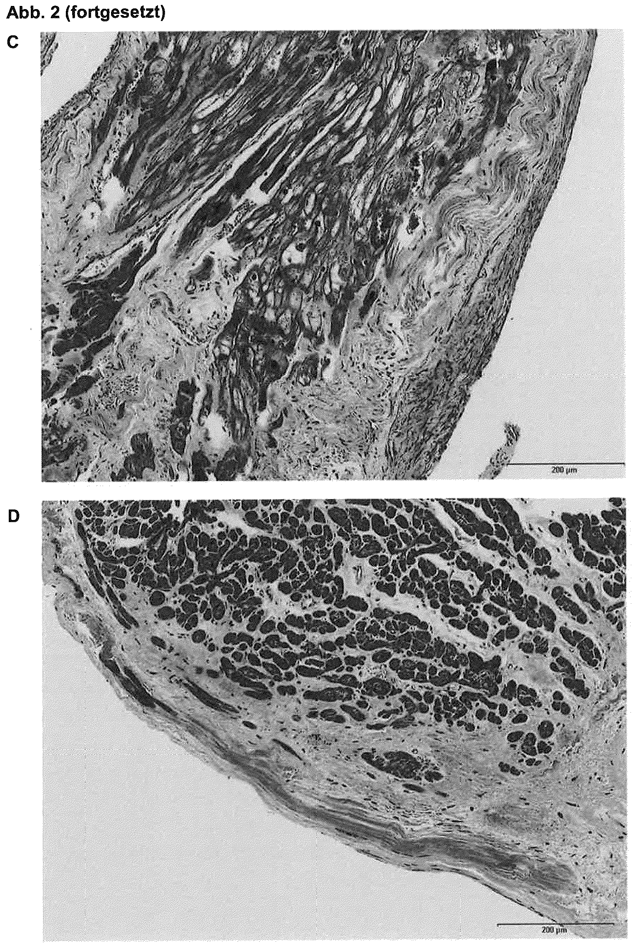

Fast alle LV-Biopsien (90/100) enthielten Endokard. Was die Verteilung der Myokardfibrose in allen endomyokardialen Biopsien betrifft, so fanden die Erfinder überwiegend interstitielle Fibrose(72%), gefolgt von subendokardialer Fibrose (19%) und Ersatzfibrose (9%). Histologische Beispiele aus vier LV-Biopsien sind in Abb. 2 dargestellt.

## Reverses LV Remodelling nach 6 Monaten Nachbeobachtung

Nach 6 Monaten waren 14/100 TAVI-Patienten verstorben, und 19/100 waren nicht in der Lage oder weigerten sich, den klinischen Besuch durchzuführen. So konnte bei 67 Patienten (29 MF+ und 38 MF-) eine vollständige 6-Monats-Visite durchgeführt werden. Die folgenden Analysen werden sich ausschließlich auf gepaarte Beobachtungen bei diesen Patienten stützen. Allerdings muss eine positive Selektionsverzerrung durch Tod oder Immobilität der kränksten Patienten postuliert werden.

Um die Abhängigkeit des reversen linksventrikulären Remodellings von der Fibroselast zu analysieren, unterteilten die Erfinder in Patienten mit einer Ausgangs-Fibrose über (MF+) und unter (MF-) dem Median von 11%. Bezüglich der Gesamtkohorte war eine signifikante GLS-Verbesserung (MF+: -13,3±4,5 vs. -16,1±3,0, P=0,006; MF-: -16,2±4,4 vs. -17,5±2,9, P=0,04) und LVMI-Reduktion (MF+: 156,4±134,1 vs. 134,1±26,4, P=0,0001; MF-: 141,2±39,8 vs. 124,2 ±32,6, P=0,002) sowohl bei MF+- als auch bei MF- Patienten vorhanden, während die Veränderungen des LVEDV nicht signifikant waren.

Die Erfinder untersuchten dann beide AS-Subgruppen mit reduzierter Ausgangs- EF und konsekutiv größtem pathologischen Remodeling (LF-HG + LEF-LG AS Patienten, n=25). Bei den 14 MF+-Patienten war die Ausgangs-EF signifikant niedriger (P=0,046) und die EF-Erholung zum Zeitpunkt der Entlassung schlechter (MF+: 33±10 bei BL vs. 41 ±10% bei Entlassung, P=0,02; MF-: 40±4 bei BL vs. 50±7% bei Entlassung, P=0,0006), aber die EF-Werte glichen sich nach 6 Monaten in den beiden Gruppen an (49±7% in MF+ vs. 50±10% in MF-, P=0,77) (Abb. 3 a). Im Vergleich zum Ausgangsbefund zeigten sowohl MF+- als auch MF-Patienten eine signifikante LVMI-Reduktion nach 6 Monaten (MF+: 166±41 vs. 141±24 g/m$^2$, P=0,02; MF-: 171±39 vs. 137±34 g/m$^2$, P=0,007), aber die LVMI-Reduktion war in der MFGruppe stärker ausgeprägt. Ebenso konnte eine signifikante Reduktion des LVEDV nur bei MF--Patienten beobachtet werden (MF+: 125±31 bei BL vs. 109±31 ml nach 6 Monaten, P=0,20; MF-: 102±30 bei BL gegenüber 85±38 ml nach 6 Monaten, P=0,02) (Abb. 3b+c). Zusammenfassend lässt sich sagen, dass die Normalisierung der LV-Geometrie und -Funktion bei MF+-Patienten verzögert, aber nicht per se verhindert wurde.

## Entwicklung klinischer Herzinsuffizienz-Parameter nach 6 Monaten

In der gesamten Kohorte konnten signifikante Verbesserungen sowohl bei MF+- als auch bei MF- Patienten in Bezug auf die Gehstrecke im 6mwt (MF+: 193±117 vs. 252±137 m, P=0,006; MF-: 254±115 vs. 254±115). 276±104 m, P=0,01), MLHFQ-Punkte (MF+: 37±17 vs. 24±19 Punkte, P<0,0001; MF-: 33±17 vs. 26±19 Punkte, P=0,02) und NT-proBNP-Spiegel (MF+ 3442±4076 vs. 1884±2877 pg/ml, P=0,02; MF-: 2305±2747 vs. 952±1162 pg/ml, P=0.005) beobachtet werden. Die Verbesserung der NYHA-Klasse im Vergleich zum Ausgangsbefund war nur bei MF- -Patienten signifikant (MF-: P für Trend 0,02; MF+: P für Trend 0,09; Abb. 6). Zusammenfassend lässt sich sagen, dass sowohl MF+- als auch MF-Patienten von der TAVI klinisch profitiert hatten, aber die klinischen Herzinsuffizienzparameter sich nach 6 Monaten noch nicht zwischen den Gruppen angeglichen hatten.

## Gesamt- und kardiovaskuläre Mortalität bei der Nachsorge

Die prozedurale Mortalität betrug 0%. Die Nachbeobachtungszeit nach TAVI lag für jeden einzelnen Patienten zwischen 6 Monaten und 2 Jahren (Mittelwert 11 Monate). Während der Nachbeobachtung traten 22 Todesfälle auf: 6/40 NEF-HG Patienten (15%), 3/14 LEF-HG Patienten (21%), 8/26 LEF-LG Patienten (31%) und 4/16 PLF-LG Patienten (25%) starben. Unter diesen wurden 14 Todesursachen als kardiovaskulär eingestuft: 1/40 (2,5%) bei NEF-HG AS (Endokarditis), 2/14 (14%) bei LEF-HG AS (2 unerwartete plötzliche Todesfälle), 7/26 (27%) bei LEF-LG AS (Herzinsuffizienz in 2 Fällen, dokumentierte Arrhythmie bei 4 Patienten, 1 unerwarteter plötzlicher Tod) und 4/16 (25%) bei PLF-LG AS (2 unerwartete plötzliche Todesfälle, 1 Tod nach Herzoperation aufgrund einer hochgradigen Trikuspidalklappeninsuffizienz, 1 Fall von Endokarditis). Nicht-kardiale Todesfälle traten auf infolge maligner Erkrankungen (n=4; 3 NEF-HG, 1 LEF-LG), Sepsis nach Magenkrebsoperation (n=1, NEF-HG), Aspiration (n=1, NEF-HG), Nierenversagen mit Ablehnung einer Dialysetherapie (n=1, NEF-HG) und Lungenentzündung mit Ablehnung der Intensivpflegetherapie (n=1, LEF-HG).

TAVI-Patienten zeichnen sich im Allgemeinen durch ein fortgeschrittenes Alter und eine hohe Belastung durch Komorbiditäten aus, die beide wesentlich zur Gesamtmortalität beitragen, während die Behandlung der Aortenklappenstenose selbst hauptsächlich die kardiovaskuläre Mortalität beeinflusst. Daher haben die Erfinder die kardiovaskuläre Mortalität als primären und die Gesamtmortalität als sekundären Endpunkt gewählt.

## Prädiktoren der kardiovaskulären Mortalität im Verlauf nach TAVI

Die kardiovaskuläre Mortalität war in der univariaten Analyse signifikant mit dem hämodynamischen Subtyp der Aortenklappenstenose assoziiert. Mit NEF-HG AS als Referenz war die kardiale Mortalität bei LEF-LG AS (HR 3,28; P=0,006) und bei PLF-LG AS (HR 2,12; P=0,01) signifikant höher. Auch bei LEF-HG AS (HR 6,04; P=0,09) war ein starker Trend zu höherer kardialer Mortalität zu verzeichnen. In der univariaten Analyse erwies sich die myokardiale Fibrose oberhalb des Medians als Risikofaktor mit der höchsten Hazard Ratio zur Prädiktion kardiovaskulärer Mortalität (HR 17,3, P=0,0001). Kaplan-Meier-Kurven für die kardiovaskuläre Mortalität bei Patienten mit Myokardfibrose unterhalb und oberhalb des Medians sind in Abb. 4a dargestellt. Alle univariat signifikanten basisdemographischen Charakteristika sowie Alter und Geschlecht wurden in ein Cox Proportional Hazards Model eingefügt. Dabei konnten fünf unabhängige Prädiktoren von kardialer Mortalität während der Nachbeobachtung identifiziert werden (siehe auch Forest-Plot in Abb. 5):

weibliches Geschlecht (HR 14,1; P=0.04), AnstiegLVEDV pro mL (HR 1,03; P=0.02), periphere arterielle Verschlusskrankheit (HR 41,3; P=0,001), Vorhofflimmern (HR 71,8; P=0.001) und Myokardfibrose ≥11% (HR 27,4; P=0,01). Diabetes und AS Subtyp verloren ihre Signifikanz in Anwesenheit der Myokardfibrose im Modell.

### Prädiktoren für der Gesamtmortalität nach TAVI

Kaplan-Meier-Kurven für die Gesamtmortalität bei Patienten mit Myokardfibrose unterhalb und oberhalb des Medians sind in Abb. 4b dargestellt. In der univariaten Analyse war die Myokardfibrose ebenfalls ein signifikanter Prädiktor der Gesamtmortalität nach TAVI (HR 2,8, CI 1,2-6,6, p=0,02). Allerdings erwiesen sich in der multivariaten Analyse nur die periphere arterielle Verschlusskrankheit und Vorhofflimmern als unabhängige Prädiktoren für die Gesamtmortalität (Forest-Plot dargestellt in Abb. 7).

### Diskussion

Ziel der vorliegenden Studie war, die myokardiale Fibrose mit hämodynamischen Aortenstenose-Phänotypen und linksventrikulärem Remodelling vor TAVI einerseits sowie mit linksventrikulärer Erholung sowie klinischem Verlauf nach TAVI andererseits zu verknüpfen.

### Die wichtigsten Ergebnisse sind:

1. Myokardfibrose oberhalb des Medians vor TAVI ist ein unabhängiger Prädiktor kardiovaskulärer Mortalität im Langzeitverlauf nach TAVI.
2. Die Höhe der histologischen myokardialen Fibrose korreliert mit dem Ausmaß des pathologischen linksventrikulären Remodellings und mit der klinischen Herzinsuffizienz. Vor TAVI ist die Fibrose oberhalb des Medians mit einer geringeren EF und geringeren GLS-Werten, mit einem größeren linksventrikulären end-diastolischen Volumen und einer höheren LV-Masse, mit einer höheren NYHA-Klasse und einer geringeren Gehstrecke im 6mwt assoziiert. Dementsprechend ist die Fibrose-Last bei LEF-HG- und LEF-LG-Patienten am höchsten.
3. Obwohl die Myokardfibrose über dem Median eine Verzögerung bei der Normalisierung der LV-Geometrie und -Funktion verursacht, können auch bei MF+-Patienten 6 Monate nach TAVI ein signifikantes reverses linksventrikuläres Remodelling und ein klinischer Nutzen der Prozedur beobachtet werden.

### Beurteilung der Myokardfibrose

Nach unserem Kenntnisstand ist dies die erste Studie an TAVI-Patienten, die den Einfluss der histologischen Myokardfibrose auf das pathologische linksventrikuläre Remodelling sowie auf das postinterventionelle reverse Remodelling und das Langzeitüberleben in verschiedenen AS-Subtypen untersucht. Die biopsiebasierte Analyse der Fibrose wurde bisher bei Patienten durchgeführt, die eine chirurgische Aortenklappenimplantation (SAVR) (Ref. 2-5, 10) erhielten, wobei sich die basisdemographischen Charakteristika dieser Patienten jedoch von denen der heutigen TAVI-Patienten deutlich unterscheiden. Darüber hinaus deuten jüngste Daten darauf hin, dass das Outcome der Patienten nach SAVR und TAVI (Ref. 11) unterschiedlich sein kann, und daher ist die Analyse der zugrunde liegenden pathophysiologischen Prozesse möglicherweise nicht vom einen Verfahren auf das andere übertragbar.

### Übergang in die Herzinsuffizienz

Um die pathophysiologischen Auswirkungen der Fibrose auf alle hämodynamischen AS-Subtypen zu bewerten, haben die Erfinder die Studienteilnehmer unterteilt in Patienten mit Myokardfibrose unterhalb (MF-) und oberhalb (MF+) des Medians. Zu Studienbeginn war MF+ mit einer reduzierten systolischen LV-Funktion (nach EF und/oder GLS), mit einem erhöhten LVEDV und einem erhöhten LVMI verbunden. Parallel dazu waren MF+-Patienten in höheren NYHA-Klassen und erreichten niedrigere Geshstrecken im 6min-Gehtest. Diese Ergebnisse stimmen mit früheren Studien überein, die darauf hindeuten, dass Fibrose eine wichtige Determinante des Übergangs von der kompensierten Hypertrophie zur manifesten Herzinsuffizienz (Ref. 2-4, 10) darstellen könnte.

In Anbetracht der Unterschiede in den Basiseigenschaften und der Anamnese bei unseren Patienten mit reduzierter EF schien die LV-Dysfunktion bei der Mehrheit der LEF-HG-Patienten überwiegend durch die unbehandelte Aortenklappenstenose selbst verursacht zu sein. So scheint sich die unbehandelte NEF-HG zu einer LEF-HG AS zu entwickeln. Dementsprechend weisen LEF-HG-Patienten eine signifikant höhere Fibrose-Last auf als NEF-HG-Patienten. Bei LEF-LG-Patienten scheint dieser Prozess bei vielen Patienten durch eine koronare Herzerkrankung aggraviert zu werden, was auf eine Kombination von Ischämie und Druckbelastung als zugrunde liegende Mechanismen hindeutet. LEF-LG-Patienten zeigten dabei die höchste Fibrose-Last unter allen Subtypen.

Die Entwicklung des Phänotyps PLF-LG AS, der sich durch kleine hypertrophe Ventrikel, hohe linke Vorhofvolumina und eine hohe Prävalenz von Vorhofflimmern auszeichnet, scheint einem anderen Pathway zu folgen. Im Vergleich zu NEF-HG litten PLG-LG-Patienten unter fortgeschritteneren Herzinsuffizienz-Symptomen und zeigten häufiger Fibrose über dem Median, wie zuvor beschrieben (Ref. 10), was darauf hindeutet, dass der PLF-LG-Remodelling-Phänotyp ebenfalls maladaptiv ist.

**Topographie der Myokardfibrose in LV-Biopsien**

Treibel et al. (Ref. 9) beschrieben drei MF-Hauptmuster bei schwerer AS: verdicktes Endokard mit massiver Fibroseschicht; ein Fibrosegradient vom Subendomyokard zum mittleren Myokard mit reichlich mikroskopischen Narben; und diffuse interstitielle Fibrose mit Bändern um die Kardiomyozyten herum. Sie identifizierten die subendokardiale Fibrose überwiegend als Mikronarben. Im Gegensatz dazu zeigen unsere histologischen Analysen eher subendokardiale Fibrosebänder mit tumorähnlicher Fibroblasteninfiltration (besonders ausgeprägt z.B. in Abb. 2c), was darauf hindeutet, dass diese Art der Fibrose besonders aktiv und damit ein potenzielles Therapieziel sein kann.

**Auswirkungen der Myokardfibrose auf die post-TAVI-Ergebnisse: Reverses Remodelling und klinischer Nutzen**

Das Konzept des reversen Remodelling nach Therapie der Aortenstenose wurde in früheren echokardiographischen und MRT-Studien nachgewiesen, die eine LVMI-Regression von etwa 20-30% 6-18 Monate nach AVR (Ref. 17-20) zeigten. Wichtig ist, dass Patienten ohne Ausgangs-Fibrose (gemessen mit LGE) eine größere LVMI-Regression aufwiesen als Patienten mit Fibrose (Ref. 18). In unserer eigenen Studie beobachteten die Erfinder sechs Monate nach TAVI eine LVMI-Reduktion von etwa 15%, der bei Personen mit Myokardfibrose über und unter dem Median vorlag. Die Analyse des LVEDV zeigte jedoch ein weniger vollständiges und verzögertes reverses Remodelling bei Patienten mit einer höheren Fibrose-Last.

Sequenzielle Biopsiebefunde von Krayenbuehl et al. (Ref. 3) deuten darauf hin, dass die frühe Reduktion der linksventrikulären Masse nach chirurgischem Aortenklappenersatz überwiegend durch die Regression der myokardialen zellulären Hypertrophie verursacht wird, während ein signifikanter Rückgang der linksventrikulären Myokardfibrose (wenn auch unvollständig) erst 6-7 Jahre später beobachtet werden kann. In einer kürzlich durchgeführten MRT-Studie von Treibel et al. (Ref. 13) war die mittels MRT gemessene fokale Fibrose nach einem Jahr nicht regredient, aber die diffuse interstitielle Fibrose und myokardiale zelluläre Hypertrophie schon, was von strukturellen und funktionellen LV-Verbesserungen begleitet war. Die Autoren gehen davon aus, dass diffuse interstitielle Fibrose plastisch und damit ein therapeutisches Ziel darstellen kann.

Bezüglich der Erholung der systolischen LV-Funktion bei Patienten mit verminderter Ausgangs-EF berichteten Weidemann et al. (Ref. 4) über einen EF-Anstieg nach SAVR ausschließlich bei Patienten ohne Ausgangs-Fibrose. Im Gegensatz dazu beobachteten die Erfinder eher eine verzögerte EF-Erholung bei MF+-Patienten: Während die schnelle EF-Verbesserung zum Zeitpunkt der Entlassung (die höchstwahrscheinlich eine einfache Folge der sofortigen Nachlastreduktion ist) bei MF--Patienten viel ausgeprägter war, glichen sich die EF-Werte der MF- und MF+-Patienten nach sechs Monaten an. Darüber hinaus sahen Weidemann et al. (Ref. 4) keine klinische Verbesserung bei Patienten mit schwerer Ausgangs-MF, während unsere TAVI MF+-Patienten einen klinischen Nutzen zeigten. Diese Outcome-Differenzen könnten auf die höhere Invasivität der chirurgischen AVR (Weidemann et al.) im Vergleich zu TAVI (unsere Studie) zurückzuführen sein.

Zusammenfassend lässt sich sagen, dass TAVI-Patienten im Allgemeinen das Potenzial eines reversen Remodelling und eines klinischen Nutzens aufweisen, aber die Normalisierung der LV-Geometrie und -Funktion wird bei Patienten mit höherer fibrotischer Last verzögert.

**Gesamt- und kardiovaskuläre Mortalität nach TAVI**

Studien, die die Auswirkungen der Myokardfibrose auf das Überleben nach SAVR oder TAVI untersuchen, sind selten (vgl. Ref 5, 12, 14, 15). Azevedo et al. (Ref. 5) bleibt die einzige Studie, die histologische Myokardfibrose und Überleben verbindet. Die Autoren zeigten, dass myokardiale Fibrose, gemessen entweder mittels Histopathologie oder MRT, einen unabhängigen Prädiktor der Gesamtmortalität bei 28 AS-Patienten nach chirurgischem Aortenklappenersatz darstellte.

Zwei weitere Studien evaluierten die Myokardfibrose ausschließlich durch LGE im MRT. Dweck et al. (Ref. 21) konnten zeigen, dass LGE ein unabhängiger Prädiktor der Mortalität bei 143 Patienten mit mittelschwerer oder schwerer Aortenstenose war (nur 70% erhielten einen Aortenklappenersatz). Die jüngste MRT-Studie von Musa et al. (Ref. 12) verfolgte 674 Patienten für mindestens 2 Jahre nach SAVR oder TAVI. LGE in der Ausgangs-MRT war unabhängig mit der Langzeitmortalität (2-fache Erhöhung) assoziiert.

In unserer eigenen Studie erwies sich die histopathologische Baseline-Fibrose über dem Median von 11% als univariat signifikanter Prädiktor für die Gesamtmortalität und als unabhängiger Prädiktor für die kardiovaskuläre Mortalität während der Nachbeobachtung nach TAVI. Aufgrund des fortgeschrittenen Alters und multipler Komorbiditäten beobachteten die Erfinder eine hohe Inzidenz von nicht-kardiovaskulären Todesfällen in unserer Kohorte, und unsere Studie war nicht auf die Gesamtmortalität ausgerichtet. Allerdings traten 13/14 kardiovaskulären Todesfälle (einschließlich acht plötzlicher Herztodesfälle) bei MF+-Patienten auf. Tatsächlich betrug die Mortalität bei MF+-Patienten 26,5%, während sie bei MF--Patienten nur 2% betrug.

LV-Biopsien von zwei Patienten mit kardialer Todesursache im Verlauf nach TAVI sind in Abb. 2c+d dargestellt. Bereits vor 30 Jahren wurde beschrieben, dass Aortenstenose-Patienten auch nach Aortenklappenersatz noch für einen plötzlichen Herztod prädisponiert sind, und dieser Befund scheint im Zusammenhang mit der fortgeschrittenen linksventrikulären Hypertrophie zu stehen (Ref. 22). Die Myokardfibrose könnte das fehlende Glied zwischen dem fortgeschrittenen linksventrikulären Remodelling und dem plötzlichen Herztod nach (T)AVR sein. Wie unsere Studie und frühere Publikationen zeigen, erfolgt der Klappenersatz bei schwerer AS häufig erst nach dem Auftreten eines potenziell irreversiblen linksventrikulären Remodellings durch Myokardfibrose, was die linksventrikuläre Erholung, den klinischen Nutzen und das Überleben nach (T)AVR negativ beeinflusst.

## Schlussfolgerungen

Histopathologische myokardiale Fibrose bei schwerer AS korreliert mit dem AS-Subtyp, dem Ausmaß des LV-Remodelling und der klinischen Herzinsuffizienz. Es verzögert, hemmt aber nicht per se das reverse LV-Remodelling und den klinischen Nutzen nach TAVI. Zu betonen ist, dass die Myokardfibrose oberhalb des Medians einen unabhängigen Prädiktor kardiovaskulärer Mortalität nach TAVI darstellt.

**Tabelle 1: Klinische Ausgangswerte**

| | Total cohort (n=100) | NEF-HG AS (n=40) | LEF-HG AS (n=14) | LEF-LG AS (n=26) | PLF-LG AS (n=16) | P (Vergleich aller Gruppen) |
|---|---|---|---|---|---|---|
| Alter, y | 78±7 | 78 ±7 | 78±9 | 79±6 | 81±5 | 0,37 |
| Geschlecht, weiblich, n (%) | 35 | 15 (38) | 5 (36) | 5(19)[d] | 9(56)[c] | 0,11 |
| Koronare Herzkrankheit, n (%) | 70 | 26 (65) | 9 (64) | 20(77) | 11(69) | 0,75 |
| Prior MI, n (%) | 22 | 3 (8)[c] | 2 (14)[c] | 12(46)[a,b] | 3 (19) | 0.002* |
| Prior PCI, n (%) | 36 | 11 (28)[c] | 2 (14)[c,d] | 14(54)[a,b] | 8 (50)[b] | 0,03* |
| Vorherige CABG, n (%) | 11 | 4(10) | 1 (7) | 4(15) | 1 (6) | 0,76 |
| Ischämische Kardiomyopathie, n(%) | 15 | 0[b,c] | 3 (21)[a] | 12 (46)[a,d] | 0° | <0,001* |
| Dilatative Kardiomyopathie, n (%) | 2 | 0 | 0 | 2 (8) | 0 | 0,14 |
| Vorhofflimmern, n (%) | 43 | 16 (40) | 5 (36) | 10 (38) | 11 (69) | 0,18 |
| Periphere Gefäßerkrankungen | 27 | 11 (28) | 3 (21) | 9 (35) | 2 (13) | 0,44 |
| Vorheriges zereb. ischem. Ereignis, n(%) | 19 | 8 (20) | 1 (7) | 6 (23) | 4 (25) | 0,60 |
| Chron. Lungenerkrankung, n (%) | 21 | 6 (15) | 1 (7) | 9 (35) | 5 (31) | 0,10 |
| Diabetes, n (%) | 45 | 18 (45) | 3 (21)[c] | 14 (54)[b] | 8 (50) | 0,48 |
| CKD, GFR < 60 mL/min, n (%) | 54 | 19 (48) | 6 (43) | 15 (58) | 12 (75) | 0,22 |

(fortgesetzt)

| | Total cohort (n=100) | NEF-HG AS (n=40) | LEF-HG AS (n=14) | LEF-LG AS (n=26) | PLF-LG AS (n=16) | P (Vergleich aller Gruppen) |
|---|---|---|---|---|---|---|
| Kreatinin, mg/dl | 1,23 ±0,7 | 1,26 ±1,0 | 1,09 ±0,3 | 1,27±0,4 | 1,27±0,9 | 0,90 |
| NT-proBNP, pg/ml | 4901 ±9444 | 2206 ±3411[b,c] | 10061 ±11082[a,d] | 8228 ±14712[a] | 2117±1185 b | <0,0001* |
| MLHFQ, Punkte | 38 ±18 | 34±19[d] | 42 ±17 | 40 ±14 | 46±15[a] | 0,09 |
| 6mwt Entfernung, m | 213 ±121 | 252 ±95[b,c,d] | 169 ±147[a] | 186 ±117[a] | 168 ±125[a] | 0.03* |

- a: p<0,05 vs. NEF-HG AS; b: p<0,05 vs. LEF-HG AS; c: p<0,05 vs. LEF-LG AS; d: p<0,05 vs. PLF-LG AS
- 2-Gruppen-Vergleiche: t-Test für kontinuierliche Variablen; Fisher's exact test für kategorische Variablen
- Vergleich aller 4 Gruppen: 1-Wege ANOVA für kontinuierliche Variablen; Chi-Quadrat-Test für kategorische Variablen
- MI: Myokardinfarkt; PCI: perkutane Koronarintervention; CABG: Koronararterien-Bypass-Optimierung; CKD: chronische Nierenerkrankung; MLHFQ: Minnesota Leben mit Herzinsuffizienz Fragebogen zur Lebensqualität; 6mwt: Sechs Gehminuten Entfernung

**Tabelle 2**

| | MF-(MF <11%) (n=51) | MF+ (MF≥11%) (n=49) | P |
|---|---|---|---|
| Alter, y | 80 ±6 | 77 ±7 | 0,01* |
| Geschlecht, weiblich, n (%) | 20 (39) | 15 (31) | 0,19 |
| Koronare Herzkrankheit, n (%) | 38 (75) | 32 (65) | 0,28 |
| Vorheriger Myokardinfarkt, n (%) | 11 (22) | 11 (22) | 1,0 |
| Vorhofflimmern, n (%) | 20 (39) | 23 (47) | 0,54 |
| Periphere Gefäßerkrankungen | 9 (18) | 18 (37) | 0,04* |
| Diabetes, n (%) | 14 (27) | 30 (61) | 0,001* |
| CKD, GFR < 60 mL/min, n (%) | 27 (53) | 27 (55) | 1,0 |
| NT-proBNP, pg/ml | 3142±3824 | 6620±1256 9 | 0,09 |
| 6mwt, m | 245±112 | 179±122 | 0,01* |
| NYHA III+IV, n (%) | 34 (67) | 40 (82) | 0,11 |
| AS-Subtyp: | | | 0,03* |
| NEF-HG AS, n (%) | 27/40 (68) | 13/40 (33) | 0,002* |
| LEF-HG AS, n (%) | 5/14 (36) | 9/14 (64) | 0,13 |
| LEF LG AS, n (%) | 9/26 (35) | 17/26 (65) | 0,03* |
| PLF-LG AS, n (%) | 7/16 (44) | 9/16 (56) | 0,48 |
| LV-EF, % | 55±11 | 44±17 | 0.0002* |
| Globale Langzeitdehnung, % | -16,3±4,2 | -13,1±4,7 | 0,001* |
| Schlagvolumenindex, ml/m$^2$ | 38±11 | 34±8 | 0,04* |
| LVEDV, ml | 79±33 | 107±45 | 0.0006* |
| LVMI, g/m$^2$ BSA | 142±39 | 161±43 | 0,03* |
| Exzentrische Hypertrophie, n (%) | 2 (4) | 12 (24) | 0,004* |
| Mittlerer transaortaler Gradient, mmHg | 39±15 | 36±16 | 0,44 |

(fortgesetzt)

|  | MF-(MF <11%) (n=51) | MF+ (MF≥11%) (n=49) | P |
|---|---|---|---|
| Alter, y | 80±6 | 77±7 | 0.01* |

·2-Gruppen-Vergleiche: t-Test für kontinuierliche Variablen; Fisher's exact test für kategorische Variablen
·CKD: chronische Nierenerkrankung; 6mwt: Sechs Gehminuten Entfernung; NYHA: New York Heart Assoziation; LVEDV: linksventrikuläres enddiastolisches Volumen; LVMI: linksventrikulärer Massenindex; BSA: Körperoberfläche; LV-EF: linksventrikuläre Ejektionsfraktion

**Tabelle 3**

| | Total cohort (n=100) | NEF-HG AS (n=40) | LEF-HG AS (n=14) | LEF-LG AS (n=26) | PLF-LG AS (n=16) | P (Vergleich aller Gruppen) |
|---|---|---|---|---|---|---|
| Vorhofflimmern während des Echos, n (%) | 25 | 7 (18)[d] | 4 (29) | 4 (15)[d] | 9 (56)[a,c] | 0.01* |
| LV-EF, % | 50±15 | 62±5 [b,c] | 34±11 [a,d] | 34±10 [a,d] | 59±7 [b,c] | <0.0001* |
| Global longitudial strain, % | -14,7±4,7 | -18-3±2,6 [b,c,d] | -10-1±3,7 [a,d] | -10,6±3,4 [a,d] | -16,1±2,0 [a,b,c] | <0,0001* |
| LVEDV, ml | 92±41 | 77±31 [b,c] | 128±43 [a,d] | 118±35 [a,d] | 67±32 [b,c] | <0.0001* |
| LVEDVi, ml/m² BSA | 48±21 | 40±15 [b,c] | 64±20 [a,d] | 62±20 [a,d] | 34±14 [b,c] | <0.0001* |
| Schlagvolumenindex, ml/m² | 36±10 | 43±10 [a,b,c] | 34±8 [a,d] | 30±7 [a] | 29±4 [a,b] | <0.0001* |
| LAVI, ml/m² BSA | 52±17 | 48±14 | 64±25 | 50±13 | 55±17 | 0.02* |
| LVMI, g/m² BSA | 151±42 | 146±38 [b] | 181±41 [a,d] | 163±43 [d] | 128±31 [b,c] | 0.002* |
| LVMI/ LVEDVi, g/ml | 3,50±1,16 | 3,94±1,18 [b,c] | 2,94±0,74 [a,d] | 2,74±0,68 [a,d] | 4,04±1,32 [b,c] | <0,0001* |
| LVEDD, mm | 47±8 | 44±7 [b,c] | 55±8 [a,d] | 52±7 [a,d] | 43±6 [b,c] | <0.0001* |
| Hinterwand, mm | 13,8±2,1 | 14,5±2,0 [c] | 13,4±2,3 | 13,2±2,2 [a] | 13,8±1,8 | 0,07 |
| Relative Wanddicke | 0,60±0,14 | 0,67±0,13 [b,c] | 0,50±0,10 [a,d] | 0,51±0,11 [a,d] | 0,64±0,07 [b,c] | <0,0001* |
| Konzentrisches Remodelling, n (%) | 10 | 4 (10) | 1 (7) | 0 [d] | 3 (19)[c] | 0,24 |
| Konzentrische Hypertrophie, n(%) | 76 | 35 (88) [b] | 8 (57) [a] | 18 (69) | 13 (81) | 0,08 |
| Exzentrische Hypertrophie, n (%) | 14 | 1 (3) [b,c] | 5 (36) [a,d] | 8 (31) [a,d] | 0 (0) [b, c] | 0.0005* |
| E/e' Mittel | 18±7 | 20±7 | 19±7 | 15±5 | 16±7 | 0,29 |
| Vmax, m/s | 3,9±0,7 | 4,5±0,5 [c,d] | 4,4±0,5 [c,d] | 3,3±0,4 [a,b] | 3,3±0,3 [a,b] | <0,0001* |
| Mittlerer Gradient, mmHg | 37±15 | 49±14 [c,d] | 45±11 [c,d] | 24±7 [a,b] | 25±5 [a,b] | <0,0001* |
| Aortenklappenöffnungsfläche (AVA), cm² | 0,74±0,17 | 0,73±0,18 | 0,62±0,12 [c,d] | 0,76±0,18 [b] | 0,77±0,13 [b] | 0,06 |
| Indexierte AVA, cm²/m² BSA | 0,39±0,09 | 0,39±0,08 [b] | 0,33±0,07 [a,c,d] | 0,40±0,08 [b] | 0,41±0,10 [b] | 0,04* |
| PAsP, mmHg | 48±16 | 45±15 | 55±19 | 46±11 | 52±21 | 0,21 |

(fortgesetzt)

| | Total cohort (n=100) | NEF-HG AS (n=40) | LEF-HG AS (n=14) | LEF-LG AS (n=26) | PLF-LG AS (n=16) | P (Vergleich aller Gruppen) |
|---|---|---|---|---|---|---|
| TAPSE, mm | 20±5 | 23±5 [b,c,d] | 19±4 a | 19±4 a | 19±4 a | 0.0005* |
| Mittlere oder schwere MR, n (%) | 41 | 11 (28) [c] | 7 (50) | 17 (65) [a] | 6 (38) | 0,02* |
| I Mittlerer oder schwerer TR, n (%) I | 32 | 8 (20) [b] | 8 (57) [a] | 8 (31) | 8 (50) | I 0,03* |

- a: p<0,05 vs. NEF-HG AS; b: p<0,05 vs. LEF-HG AS; c: p<0,05 vs. LEF-LG AS; d: p<0,05 vs. PLF-LG AS
- 2-Gruppen-Vergleiche: t-Test für kontinuierliche Variablen; Fisher's exact test für kategorische Variablen
- Vergleich aller 4 Gruppen: 1-Wege ANOVA für kontinuierliche Variablen; Chi-Quadrat-Test für kategorische Variablen
- LV-EF: linksventrikuläre Ejektionsfraktion; LVEDV: linksventrikuläres enddiastolisches Volumen; BSA: Körperoberfläche; SVI: Hubvolumenindex; LA-VI: linksatriales Volumen Index; LVMI: linksventrikulärer Massenindex; LVEDD: linksventrikulärer enddiastolischer Durchmesser; RWT: relative Wanddicke; GLS: globale Längsdehnung; vmax: maximale Aortengeschwindigkeit; AVA: Aortenklappe sind; AVAi: indexierte Aortenklappenfläche, AVA/BSA; PAsP: Lungenarterie systolischer Druck; TAPSE: tricuspid annular Ebene systolische Erhebung; MR: Mitralinsuffizienz; TR: Tricuspidale Insuffizienz

### Beispiel 2

Die Wirkung von niedrig dosiertem Hydralazin auf Myokardfibrose wurde in einem Mausmodel für Angiotensin II-(AT-II-)induzierte kardiomyozytäre Hypertrophie und kardiale Fibrose untersucht. Zusammenfassend wurde den Versuchstieren dazu täglich AT-II, bzw. AT-II und Hydralazin verabreicht. An Tag 14 der Behandlung wurde Herzgewebe entnommen und mittels Antikörperfärbung gegen Kollagen-1 der Fibrosegrad bestimmt. Weiterhin wurde der DNA-Methylierungsgrad in der RASAL 1 Region analysiert. Die zusätzliche Behandlung mit einer geringen Dosis Hydralazin (5mg/kg Körpergewicht) führt zu einer deutlichen Reduktion der Fibrose. Ebenfalls wurde nachgewiesen, dass die für fibrotisches Gewebe charakteristische Methylierung der RASAL 1 Region geringer ist.

### Behandlung der Mäuse:

AT-II: Die Induktion von kardialer Fibrose im Mausmodell wurde mittels AT-II-Applikation realisiert. Dabei wurden als profibrotische Effekte die Angiotensin-Rezeptorbindung mit konsekutiver peripherer Vasokonstriktion und Nachlasterhöhung des Herzens sowie die TGF-$\beta$1-Modulation genutzt. Dieses Modell zur Induktion von Fibrose und kardiomyozytärer Hypertrophie wurde bereits von Matsui et al., 2004 und Schellings et al., 2010 beschrieben.

AT-II und Hydralazin wurden täglich verabreicht, an Tag 14 wurde Gewebe entnommen und der Fibrosegrad, sowie der DNA-Methylierungsgrad bestimmt. Kontrolltiere wurden für dieselbe Zeitdauer der korrespondierenden Kohorte mit phosphatgepufferter Kochsalzlösung (PBS) behandelt.

### Kollagenfärbungen:

### 1) Kollagen-1 Antikörperfärbung (+DAPI)

Antikörperfärbung nach Standardprotokoll mit polyklonalem Anti-Kollagen-1-IgG (Abcam, Cambridge, UK, Katalognr.: ab34710), Verdünnung: 1:200.

### 2) MTS-Färbung

Die Masson-Trichrom-Färbung (MTS) diente im Rahmen dieser Arbeit hauptsächlich der Quantifizierung des Fibrosegrades der Mäuseherzen. Sie eignete sich dafür besonders gut, weil sich das blaue Kollagen kontrastreich von den rosaroten Kardiomyozyten abhob. Nach abgeschlossener Rehydrierung wurden die Schnitte für 15 Min. in 56 °C-warmer Bouin-Lösung nochmals fixiert. Unter fließendem Leitungswasser wurden die Präparate dann gespült, bevor sie für 5 Min. zur Zellkernfärbung in Weigert's Eisenhämatoxylin-Lösung getaucht wurden. Der Farbumschlag fand während des folgenden Spülens unter fließendem Leitungswasser statt, woraufhin dreimalig in destilliertem Wasser gewaschen wurde. Die rote Zytoplasmafärbung erfolgte mittels fünfminütiger Behandlung in Biebrich-Scharlachrot-Säurefuchsin-Lösung. Nach abermaliger dreimaliger Waschung in destilliertem Wasser wurden die Präparate in Vorbereitung der darauffolgenden Färbung mit Anilinblau für 5 Min. in eine Phosphomolybdän-/ Phosphowolframsäure-Lösung getaucht. Die anschließende 10-minütige Einwirkzeit in Anilinblau bewirkte die Anfärbung der Kollagenfasern. Eine Ausdifferenzierung der Färbung erfolgte durch das jeweils dreifache Eintauchen in 1%ige Essigsäure und destilliertes Wasser. Die Konservierung der gefärbten Schnitte erforderte eine erneute Entwässerung, die durch das jeweils dreimalige Eintauchen in 96%iges und dann in 100%iges Ethanol erfolgte. Zum Schluss wurden die Schnitte abermals mit Xylol (2x 5 Min.) behandelt und mit Entellan eingedeckt.

Repräsentative Fluoreszenzbilder sind in Abbildung 8 gezeigt.

Die prozentuale linksventrikuläre interstitielle Fläche der Kollagen-1-Färbung wurden anhand der Fluoreszenzbilder berechnet. Diese Daten sind in Abbildung 9 dargestellt.

Wie die Ergebnisse der Fluoreszenzfärbungen zeigen, wurde die durch AT-II induzierte Vergrößerung der Kollagen-1-positiven Bereiche bei Behandlung mit Hydralazin deutlich reduziert, was auf Verringerung der Fibrose hinweist.

## MeDIP:

Die Messung und der Vergleich unterschiedlicher DNA-Methylierungsgrade von Promotorbereichen spezifischer Gene setzte die Trennung methylierter DNA-Fragmente vom Rest der genomischen DNA voraus. Dies wurde durch die Methylated DNA immunoprecipitation (MeDIP) gewährleistet. Zur Durchführung diente das "Methylamp Methylated DNA Capture (MeDIP) Kit" von EpiGentek (Farmingdale, NY, USA) samt Herstellerprotokoll, nach welchem mit geringen Änderungen vorgegangen wurde. Die Methode wurde zuerst von Weber et al., 2005 beschrieben. Ihr Prinzip besteht aus der Anreicherung methylierter DNA durch die Bindung eines Antikörpers an 5mC. Ausgangsmaterial war aus Gewebe oder Vollblut extrahierte genomische DNA (s.o.). Zunächst musste sichergestellt werden, dass im weiteren Verlauf ausschließlich Probenmengen der gleichen Konzentration eingesetzt wurden, um letztendlich gemessene Mengenunterschiede tatsächlich auch auf unterschiedliche Methylierungsgrade zurückführen zu können. Daher wurden die DNA-Proben in einer Weise mit MC2-Puffer (reaction buffer) verdünnt und in 1,5 ml-Eppendorf-Gefäße überführt, dass in 108 $\mu$l Totalvolumen je 500 ng oder 1000 ng DNA (abhängig von der geringsten gemessenen Konzentration der jeweiligen Kohorte) gelöst waren. Eine weitere Voraussetzung der Methode war die Zerlegung der DNA-Stränge in ähnlich große Fragmente von 500-1000 bp. Die Probengefäße wurden zu diesem Zweck in ein Eis-Wasserbad gegeben und von unten für 10 Min. bei einer Amplitude von 20 % der Geräteleistung mit Ultraschall einer Frequenz von 20 kHz beschossen (sonication). Durch den Schall wurden im Wasser Blasen erzeugt, die bei Aussetzen der akustischen Energie kollabierten und dabei Schockwellen freisetzten, welche die DNA-Stränge brechen ließen. Um die DNA-Fragmente den 5mC-Antikörpern zugänglich zu machen, mussten sie in Einzelsträngen vorliegen. Eine anschließende zweiminütige Denaturierung bei 95 °C stellte dies sicher. In der Zwischenzeit wurde eine Probenplatte (well plate) vorbereitet, in welcher später die Bindung der 5mC-Antikörper an die methylierten DNA-Fragmente stattfinden sollte. Dafür wurden in jede Vertiefung (well) der Platte je 100 $\mu$l MC1-Puffer (antibody buffer) und 1 $\mu$l 5mC-Antikörper gegeben und die well plate mit Parafilm M bedeckt. In der nun folgenden einstündigen Inkubationszeit bei Raumtemperatur auf der Schüttelplatte konnten die Antikörper an die beschichteten Böden der wells binden. Nach Ende der Inkubation wurden die wells von der Flüssigkeit befreit und jeweils einmal mit 150 $\mu$l der MC1- und MC3-Puffer (wash buffer) gewaschen. Nun wurden jeweils 100 $\mu$l der fragmentierten und denaturierten DNA-Proben in die wells gegeben und die well plate (von Parafilm M bedeckt) für zwei Stunden bei Raumtemperatur auf die Schüttelplatte gestellt. Während dieser Zeit wurden diejenigen DNA-Fragmente von den an den Böden haftenden Antikörpern gebunden, die über 5mC verfügten (pull-down). Die unmethylierten Fragmente blieben im Puffer gelöst. Nach Ablauf der Inkubation wurden die wells dreimal mit 150 $\mu$l MC3-Puffer gewaschen, um alle nicht gebundenen 33 Fragmente zu entfernen. Jeweils 60 $\mu$l MC4-Puffer (DNA release buffer) und 1 $\mu$l Proteinase K wurden daraufhin in jedes well pipettiert und in einem Wasserbad bei 65 °C für eine Stunde inkubiert. Genauso wurde mit einem Input-1,5 ml Eppendorf-Gefäß mit 5 $\mu$l der fragmentierten DNA verfahren, das ohne pull-down bis zum Ende die gesamte genomische DNA (Input-DANN) beinhaltete und am Ende als Bezugsgröße diente. Nach erfolgter Inkubation wurde je Probe und Input eine Spin-Säule mit 100 $\mu$l MC5-Puffer (binding buffer) versehen. Zu Fällung der DNA wurden dann in jedes well und jedes Input-Gefäß 180 $\mu$l reines Ethanol gegeben, bevor der resuspendierte Inhalt dann auf die Spin-Säulen überführt wurde. Diese wurden anschließend bei 12000 U/Min. für 30 Sek. zentrifugiert. Die DNA konnte nun vom Silikagel der Spin-Säulen gebunden werden (siehe DNA-Isolation). Der Vorgang wurde zweimal mit jeweils 200 $\mu$l 90%igen Ethanols wiederholt. Zuletzt wurde ein Volumen von 20 $\mu$l MC6-Puffer (elution buffer) auf die Säule gegeben, diese bei 12000 U/Min. für 30 Sek. zentrifugiert und das Eluat in einem neuen 1,5 ml Eppendorf-Gefäß aufgefangen. Um ein ausreichendes Probenvolumen der aufgereinigten methylierten DNA zu erhalten, wurden noch 80 $\mu$l Nuklease-freies Wasser dazugegeben. Die Probengefäße konnten nun bei -20 °C zur Aufbewahrung eingefroren werden.

An die MeDIP schloss sich eine qRT-PCR zum Zweck der Amplifizierung methylierter DNA-Sequenzen an, welche durch die Wahl der Primer definiert wurden. Im Vergleich zur genomischen Input-DNA konnten damit sequenzspezifisch Rückschlüsse auf den methylierten Anteil der ursprünglich eingesetzten DNA-Probe gemacht werden.

Wie Abbildung 10 zeigt, wurde die durch AT-II induzierte RASAL1-Methylierung durch Behandlung mit Hydralazin deutlich reduziert.

**REFERENZLISTE**

1. Baumgartner H, Falk V, Bax JJ, De Bonis M, Hamm C, Holm PJ, Iung B, Lancellotti P, Lansac E, Rodriguez Munoz D, Rosenhek R, Sjogren J, Tornos Mas P, Vahanian A, Walther T, Wendler O, Windecker S and Zamorano JL. 2017 ESC/EACTS Guidelines for the management of valvular heart disease. Eur Heart J. 2017;38:2739-2791.

2. Hein S, Arnon E, Kostin S, Schonburg M, Elsasser A, Polyakova V, Bauer EP, Klovekorn WP and Schaper J. Progression from compensated hypertrophy to failure in the pressure-overloaded human heart: structural deterioration and compensatory mechanisms. Circulation. 2003;107:984-91.

3. Krayenbuehl HP, Hess OM, Monrad ES, Schneider J, Mall G and Turina M. Left ventricular myocardial structure in aortic valve disease before, intermediate, and late after aortic valve replacement. Circulation. 1989;79:744-55.

4. Weidemann F, Herrmann S, Stork S, Niemann M, Frantz S, Lange V, Beer M, Gattenlohner S, Voelker W, Ertl G and Strotmann JM. Impact of myocardial fibrosis in patients with symptomatic severe aortic stenosis. Circulation. 2009;120:577-84.

5. Azevedo CF, Nigri M, Higuchi ML, Pomerantzeff PM, Spina GS, Sampaio RO, Tarasoutchi F, Grinberg M and Rochitte CE. Prognostic significance of myocardial fibrosis quantification by histopathology and magnetic resonance imaging in patients with severe aortic valve disease. J Am Coll Cardiol. 2010;56:278-87.

6. Kappetein AP, Head SJ, Genereux P, Piazza N, van Mieghem NM, Blackstone EH, Brott TG, Cohen DJ, Cutlip DE, van Es GA, Hahn RT, Kirtane AJ, Krucoff MW, Kodali S, Mack MJ, Mehran R, Rodes-Cabau J, Vranckx P, Webb JG, Windecker S, Serruys PW and Leon MB. Updated standardized endpoint definitions for transcatheter aortic valve implantation: the Valve Academic Research Consortium-2 consensus document. Eur Heart J. 2012;33:2403-18.

7. Lang RM, Badano LP, Mor-Avi V, Afilalo J, Armstrong A, Ernande L, Flachskampf FA, Foster E, Goldstein SA, Kuznetsova T, Lancellotti P, Muraru D, Picard MH, Rietzschel ER, Rudski L, Spencer KT, Tsang W and Voigt JU. Recommendations for cardiac chamber quantification by echocardiography in adults: an update from the American Society of Echocardiography and the European Association of Cardiovascular Imaging. European heart journal cardiovascular Imaging. 2015;16:233-70.

8. Mewton N, Liu CY, Croisille P, Bluemke D and Lima JA. Assessment of myocardial fibrosis with cardiovascular magnetic resonance. J Am Coll Cardiol. 2011;57:891-903.

9. Treibel TA, Lopez B, Gonzalez A, Menacho K, Schofield RS, Ravassa S, Fontana M, White SK, DiSalvo C, Roberts N, Ashworth MT, Diez J and Moon JC. Reappraising myocardial fibrosis in severe aortic stenosis: an invasive and noninvasive study in 133 patients. Eur Heart J. 2018;39:699-709.

10. Herrmann S, Stork S, Niemann M, Lange V, Strotmann JM, Frantz S, Beer M, Gattenlohner S, Voelker W, Ertl G and Weidemann F. Low-gradient aortic valve stenosis myocardial fibrosis and its influence on function and outcome. J Am Coll Cardiol. 2011;58:402-12.

11. Mack MJ, Leon MB, Thourani VH, Makkar R, Kodali SK, Russo M, Kapadia SR, Malaisrie SC, Cohen DJ, Pibarot P, Leipsic J, Hahn RT, Blanke P, Williams MR, McCabe, JM, Brown DL, Babaliaros V, Goldman S, Szeto WY, Genereux P, Pershad A, Pocock SJ, Alu MC, Webb JG and Smith CR. Transcatheter Aortic-Valve Replacement with a Balloon-Expandable Valve in Low-Risk Patients. The New England Journal of Medicine. 2019;380:1695-1705.

12. Musa TA, Treibel TA, Vassiliou VS, Captur G, Singh A, Chin C, Dobson LE, Pica S, Loudon M, Malley T, Rigolli M, Foley JRJ, Bijsterveld P, Law GR, Dweck MR, Myerson, SG, McCann GP, Prasad SK, Moon JC and Greenwood JP. Myocardial Scar and Mortality in Severe Aortic Stenosis. Circulation. 2018;138:1935-1947.

13. Treibel TA, Kozor R, Schofield R, Benedetti G, Fontana M, Bhuva AN, Sheikh A, Lopez B, Gonzalez A, Manisty C, Lloyd G, Kellman P, Diez J and Moon JC. Reverse Myocardial Remodeling Following Valve Replacement in Patients With Aortic Stenosis. J. Am Coll Cardiol. 2018;71:860-871.

14. Chen H, Zeng J, Liu D and Yang Q. Prognostic value of late gadolinium enhancement on CMR in patients with severe aortic valve disease: a systematic review and metaanalysis. Clinical radiology. 2018;73:983.e7-983.e14.

15. Lee H, Park JB, Yoon YE, Park EA, Kim HK, Lee W, Kim YJ, Cho GY, Sohn DW, Greiser A and Lee SP. Noncontrast Myocardial T1 Mapping by Cardiac Magnetic Resonance Predicts Outcome in Patients With Aortic Stenosis. JACC Cardiovascular Imaging. 2018;11:974-983.

16. Bing R, Cavalcante JL, Everett RJ, Clavel MA, Newby DE and Dweck MR. Imaging and Impact of Myocardial Fibrosis in Aortic Stenosis. JACC Cardiovascular Imaging. 2019;12:283-296.

17. Monrad ES, Hess OM, Murakami T, Nonogi H, Corin WJ and Krayenbuehl HP. Time course of regression of left ventricular hypertrophy after aortic valve replacement. Circulation. 1988;77:1345-55.

18. Dobson LE, Musa TA, Uddin A, Fairbairn TA, Swoboda PP, Erhayiem B, Foley J, Garg P, Haaf P, Fent GJ, Malkin CJ, Blackman DJ, Plein S and Greenwood JP. Acute Reverse Remodelling After Transcatheter Aortic Valve Implantation: A Link Between Myocardial Fibrosis and Left Ventricular Mass Regression. The Canadian journal of cardiology. 2016;32:1411-1418.

19. Lamb HJ, Beyerbacht HP, de Roos A, van der Laarse A, Vliegen HW, Leujes F, Bax JJ and van der Wall EE. Left ventricular remodeling early after aortic valve replacement: differential effects on diastolic function in aortic valve stenosis and aortic regurgitation. J Am Coll Cardiol. 2002;40:2182-8.

20. Rost C, Korder S, Wasmeier G, Wu M, Klinghammer L, Flachskampf FA, Daniel WG and Voigt JU. Sequential changes in myocardial function after valve replacement for aortic stenosis by speckle tracking echocardiography. European journal of echocardiography : the journal of the Working Group on Echocardiography of the European Society of Cardiology. 2010;11:584-9.

21. Dweck MR, Joshi S, Murigu T, Alpendurada F, Jabbour A, Melina G, Banya W, Gulati A, Roussin I, Raza S, Prasad NA, Wage R, Quarto C, Angeloni E, Refice S, Sheppard M, Cook SA, Kilner PJ, Pennell DJ, Newby DE, Mohiaddin RH, Pepper J and Prasad SK. Midwall fibrosis is an independent predictor of mortality in patients with aortic stenosis. J Am Coll Cardiol. 2011;58:1271-9.

22. Foppl M, Hoffmann A, Amann FW, Roth J, Stulz P, Hasse J, Gradel E and Burckhardt D. Sudden cardiac death after aortic valve surgery: incidence and concomitant factors. Clinical cardiology. 1989;12:202-7.

23. Ravassa S, Trippel T, Bach D, Bachran D, González A, López B, Wachter R, Hasenfuss G, Delles C, Dominiczak AF, Pieske B, Diez J, Edelmann F. Biomarker based phenotyping of myocardial fibrosis identifies patients with heart failure with preserved ejection fraction resistant to the beneficial effects of spironolactone: results from the Aldo-DHF trial. Eur J Heart Fail 2018;20:1290-1299.

24. Tampe B, Tampe D, Zeisberg EM, Müller GA, Bechtel-Walz W, Koziolek M, Kalluri R, Zeisberg M. Induction of Tet3-dependent epigenetic remodeling by lowdose hydralazine attenuates progression of chronic kidney disease. EBioMedicine 2015;2:19-36.

25. Timbrell JA, Harland SJ, Facchini V. Polymorphic acetylation of hydralazine. Clin. Pharmacol Ther. 1980 Sep;28(3):350-355.

26. Ruiz JD, Martinez C, Anderson K, Gross M, Lang NP, Garcia-Martin E, Agundez JAG. The Differential Effect of NAT2 Variant Alleles Permits Refinement in Phenotype Inference and Identifies a Very Slow Acetylation Genotype. PLOS ONE, 2012, 7(9), e44629.

27. Garces-Eisele SJ, Cedillo-Cavallo B, Reyes-Nunez V, Estrada-Martin L, Vazquez-Perez R, Juarez-Calderon M, Guzman-Garcia MO, Duenas-Gonzalez A, Ruiz-Argüelles A. Genetic selection of volunzteers and concomitant dose adjustment leads to comparable hydralazine/valproate exposure. Journal of Clinical Pharmacy and Therapeutics, 2014, doi: 10.1111/jcpt.12155.

28. Sawhney N, Hassankhani A, Greenberg BH. Calcific Aortic Stenosis in the Elderly: A Brief Overview. The American Journal of Geriatric Cardiology 2003; 12(3): 178-182.

29. Mangla A, Gupta S. Vascular complications post-transcatheter aortic valve procedures. Indian Heart Journal, 2016; 68(5): 724-731.

30. Lacolley P, Safar ME, Lucet B, Ledudal K, Labat C, Benetos A. Prevention of Aortic and Cardiac Fibrosis by Spironolactone in Old Normotensive Rats, J Am Coll Cardiol, 2001, 37(2), 662-667.

31. Cezar MDM, Damatto RL, Pagan LU, Lima ARR, Martinez PF, Bonomo C, Rosa CM, Campos DHS, Cicogna AC, Gomes MJ, Oliveira-Jr SA, Blotta DA, Okoshi MP, Okoshi K. Early Spironolactone Treatment Attenuates Heart Failure Development by Improving Myocardial Function and Reducing Fibrosis in Spontaneously Hypertensive Rats, Cell Physiol Biochem, 2015, 36, 1453-1466.

32. Ponikowski et al., 2016 ESC Guidelines for the diagnosis and treatment of acute and chronic heart failure, Eur Heart J, 2016, ehw128.

33. Xu X, Tan X, Tampe B, Nyamsuren G, Liu X, Maier LS, Sossalla S, Kalluri R, Zeisberg M, Hasenfuss G, Zeisberg EM. Epigenetic balance of aberrant Rasal1 promoter methylation and hydroxymethylation regulates cardiac fibrosis. Cardiovascular Res, 2015, 105, 279-291.

34. Zeisberg EM, Zeisberg M. A Rationale for Epigenetic Repurposing of Hydralazine in Chronic Heart and Kidney Failure. J Clin Epigenet, 2016, 2(1), 3.

35. Puls M, Beuthner BE, Topci R, Vogelgesang A, Bleckmann A, Sitte M, Lange T, Backhaus SJ, Schuster A, Seidler T, Kutschka I, Toischer K, Zeisberg EM, Jacobshagen C, Hasenfuß G. Impact of myocardial fibrosis on left ventricular remodelling, recovery, and outcome after transcatheter aortic valve implantation in different haemodynamic subtypes of severe aortic stenosis. Eur Heart J, 2020, 41, 1903-1914.

36. Matsui Y, Jia N, Okamoto H, Kon S, Onozuka H, Akino M, Liu L, Morimoto J, Rittling SR, Denhardt D, et al. (2004): Role of osteopontin in cardiac fibrosis and remodeling in angiotensin II-induced cardiac hypertrophy. Hypertension 43, 1195-1201.

37. Schellings MWM, Vanhoutte D, Almen GC van, Swinnen M, Leenders JJG, Kubben N, Leeuwen REW van, Hofstra L, Heymans S, Pinto YM (2010): Syndecan-1 Amplifies Angiotensin II-Induced Cardiac Fibrosis. Hypertension 55, 249-256.

38. Weber M, Davies JJ, Wittig D, Oakeley EJ, Haase M, Lam WL, Schübeler D (2005): Chromosome-wide and promoter-specific analyses identify sites of differential DNA methylation in normal and transformed human cells.

**Patentansprüche**

1. Anti-Fibrose-Mittel zur Anwendung in der Behandlung und/oder Prävention von Patienten mit Aortenstenose, die einer Katheter-basierten Aortenklappenimplantation (transcatheter aortic valve implantation; TAVI) unterzogen werden, wobei das Anti-Fibrose-Mittel eine Kombination von (i) Spironolacton und (ii) mindestens einem von Hydralazin und Dihydralazin ist und wobei die Patienten **dadurch gekennzeichnet sind, dass** sie eine myokardiale Fibrose aufweisen, die gleich oder höher als ein klinisch signifikanter Schwellenwert der Querschnittsfläche des Myokard ist.

2. Anti-Fibrose-Mittel zur Anwendung in der Nachbehandlung von Patienten mit Aortenstenose, die einer Katheter-basierten Aortenklappenimplantation (transcatheter aortic valve implantation; TAVI) unterzogen wurden, wobei das Anti-Fibrose-Mittel eine Kombination von (i) Spironolacton und (ii) mindestens einem von Hydralazin und Dihydralazin ist und wobei die Patienten **dadurch gekennzeichnet sind, dass** sie eine myokardiale Fibrose aufweisen, die gleich oder höher als ein klinisch signifikanter Schwellenwert der Querschnittsfläche des Myokard ist.

3. Anti-Fibrose-Mittel zur Anwendung nach Anspruch 1 oder 2, wobei der klinisch signifikante Schwellenwert ein Wert zwischen 6 % und 85 % ist, bevorzugt ein Wert zwischen 7 % und 70%, stärker bevorzugt ein Wert zwischen 8 % und 60%, stärker bevorzugt ein Wert zwischen 9 % und 50 %, stärker bevorzugt ein Wert zwischen 10 % und 40 %, stärker bevorzugt ein Wert zwischen 10 % und 30%, stärker bevorzugt ein Wert zwischen 11 % und 20 %, wie beispielsweise 11 %, 12 %, 13 %, 14 %, 15 %, 16 %, 17 %, 18 %, 19 %, oder 20 %.

4. Anti-Fibrose-Mittel zur Anwendung nach einem beliebigen der Ansprüche 1-3, wobei der klinisch signifikante Schwellenwert 11 % beträgt.

5. Anti-Fibrose-Mittel zur Anwendung nach einem beliebigen der Ansprüche 1-4, wobei die prozentuale myokardiale Fibrose mittels Myokardbiopsie und einer histologischen Analyse, bevorzugt nach Masson-Trichrom-Färbung, bestimmt wird.

6. Anti-Fibrose-Mittel zur Anwendung nach einem beliebigen der Ansprüche 1-5, wobei die Myokardbiopsie eine Myokardbiopsie des linken Ventrikels ist.

7. Anti-Fibrose-Mittel zur Anwendung nach einem beliebigen der Ansprüche 1-6, wobei die prozentuale myokardiale Fibrose zusätzlich oder alternativ mittels kardialer Magnetresonanztomographie (MRT) bestimmt wird, insbesondere mittels Bildgebung durch späte Gadoliniumanreicherung und/oder T1-Mapping.

8. Anti-Fibrose-Mittel zur Anwendung nach einem beliebigen der Ansprüche 1-7, wobei der Patient eine Aortenstenose des Subtyps PLF-LG AS oder des Subtyps LEF-LG AS aufweist;
insbesondere wobei der Patient eine Aortenstenose des Subtyps PLF-LG AS aufweist.

9. Anti-Fibrose-Mittel zur Anwendung nach einem beliebigen der Ansprüche 1-8 wobei das Anti-Fibrose-Mittel eine Kombinationstherapie mit (i) Spironolacton und (ii) Hydralazin ist.

10. Anti-Fibrose-Mittel zur Anwendung nach einem beliebigen der Ansprüche 1-9, wobei das Hydralazin oder Dihydralazin in einer Konzentration verabreicht wird, die niedriger ist als für die Hypertoniebehandlung, insbesondere wobei die Konzentration so niedrig ausgewählt wird, dass sie eine demethylierende Wirkung aufweist.

11. Anti-Fibrose-Mittel zur Anwendung nach Anspruch 9 oder Anspruch 10 , wobei das Hydralazin oder Dihydralazin an schnelle Metabolisierer in einer Dosis im Bereich von 2 x 15,1 mg pro Tag bis 2 x 25 mg pro Tag verabreicht wird, und an langsame Metabolisierer in einer Dosis im Bereich von 2 x 5 mg pro Tag bis 2 x 15 mg pro Tag verabreicht wird.

12. Anti-Fibrose-Mittel zur Anwendung nach einem beliebigen der Ansprüche 9-11, wobei das Hydralazin oder Dihydralazin an schnelle Metabolisierer in einer Dosierung von 2 x 25 mg pro Tag verabreicht wird, und an langsame Metabolisierer in einer Dosierung von 2 x 12,5 mg pro Tag verabreicht wird.

**Claims**

1. Anti-fibrosis agent for use in the treatment and/or the prevention of patients having aortic stenosis undergoing transcatheter aortic valve implantation (TAVI), wherein the anti-fibrosis agent is a combination of (i) spironolactone and (ii) at least one of hydralazine and dihydralazine, and wherein the patients are **characterized in that** they have myocardial fibrosis equal to or greater than a clinically significant threshold value of the cross-sectional area of the myocardium.

2. Anti-fibrosis agent for use in the follow-up treatment of patients having aortic stenosis who have undergone transcatheter aortic valve implantation (TAVI), wherein the anti-fibrosis agent is a combination of (i) spironolactone and (ii) at least one of hydralazine and dihydralazine, and wherein the patients are **characterized in that** they have myocardial fibrosis equal to or greater than a clinically significant threshold value of the cross-sectional area of the myocardium.

3. Anti-fibrosis agent for use according to claim 1 or 2, wherein the clinically significant threshold value is a value between 6% and 85%, preferably a value between 7% and 70%, more preferably a value between 8% and 60%, more preferably a value between 9% and 50%, more preferably a value between 10% and 40%, more preferably a value between 10% and 30%, more preferably a value between 11% and 20%, such as 11%, 12%, 13%, 14%, 15%, 16%, 17%, 18%, 19% or 20%.

4.  Anti-fibrosis agent for use according to any one of claims 1-3, wherein the clinically significant threshold value is 11%.

5.  Anti-fibrosis agent for use according to any one of claims 1-4, wherein the percentage of myocardial fibrosis is determined by means of myocardial biopsy and histological analysis, preferably after Masson's trichrome staining.

6.  Anti-fibrosis agent for use according to any one of claims 1-5, wherein the myocardial biopsy is a myocardial biopsy of the left ventricle.

7.  Anti-fibrosis agent for use according to any one of claims 1-6, wherein the percentage of myocardial fibrosis is additionally or alternatively determined by means of cardiac magnetic resonance imaging (MRI), in particular by means of imaging by late gadolinium enhancement and/or T1 mapping.

8.  Anti-fibrosis agent for use according to any one of claims 1-7, wherein the patient has an aortic stenosis of the PLF-LG AS subtype or the LEF-LG AS subtype; in particular wherein the patient has an aortic stenosis of the PLF-LG AS subtype.

9.  Anti-fibrosis agent for use according to any one of claims 1-8, wherein the anti-fibrosis agent is a combination therapy with (i) spironolactone and (ii) hydralazine.

10. Anti-fibrosis agent for use according to any one of claims 1-9, wherein the hydralazine or dihydralazine is administered in a concentration lower than for hypertension treatment, in particular wherein the concentration is selected to be low enough to have a demethylating effect.

11. Anti-fibrosis agent for use according to claim 9 or claim 10, wherein the hydralazine or dihydralazine is administered to fast metabolizers at a dose in the range of 2 x 15.1 mg per day to 2 x 25 mg per day, and to slow metabolizers at a dose in the range of 2 x 5 mg per day to 2 x 15 mg per day.

12. Anti-fibrosis agent for use according to any one of claims 9-11, wherein the hydralazine or dihydralazine is administered to fast metabolisers at a dosage of 2 x 25 mg per day, and to slow metabolizers at a dosage of 2 x 12.5 mg per day.

**Revendications**

1.  Agent antifibrotique destiné à être utilisé dans le traitement et/ou la prévention de patients atteints de sténose aortique qui sont soumis à une implantation de valve aortique par voie percutanée (transcatheter aortic valve implantation ; TAVI) , l'agent antifibrotique étant une combinaison de (i) spironolactone et (ii) au moins l'un des composés hydralazine et dihydralazine, les patients étant **caractérisés en ce qu'**ils présentent une fibrose myocardique égale ou supérieure à une valeur seuil cliniquement significative de la surface de section transversale du myocarde.

2.  Agent antifibrotique destiné à être utilisé dans le traitement de suivi de patients atteints de sténose aortique qui ont subi une implantation de valve aortique par voie percutanée (transcatheter aortic valve implantation ; TAVI) , l'agent antifibrotique étant une combinaison de (i) spironolactone et (ii) au moins l'un des composés hydralazine et dihydralazine, les patients étant **caractérisés en ce qu'**ils présentent une fibrose myocardique égale ou supérieure à une valeur seuil cliniquement significative de la surface de section transversale du myocarde.

3.  Agent antifibrotique destiné à être utilisé selon la revendication 1 ou 2, dans lequel la valeur seuil cliniquement significative est une valeur comprise entre 6 % et 85 %, de préférence une valeur comprise entre 7 % et 70 %, plus préférablement une valeur comprise entre 8 % et 60 %, plus préférablement une valeur comprise entre 9 % et 50 %, plus préférablement une valeur comprise entre 10 % et 40 %, plus préférablement une valeur comprise entre 10 % et 30 %, plus préférablement une valeur comprise entre 11 % et 20 %, telle que par exemple 11 %, 12 %, 13 %, 14 %, 15 %, 16 %, 17 %, 18 %, 19 % ou 20 %.

4.  Agent antifibrotique destiné à être utilisé selon l'une quelconque des revendications 1 à 3, dans lequel la valeur seuil cliniquement significative est de 11 %.

5. Agent antifibrotique destiné à être utilisé selon l'une quelconque des revendications 1 à 4, dans lequel le pourcentage de fibrose myocardique est déterminé par biopsie myocardique et analyse histologique, de préférence après coloration au trichrome de Masson.

6. Agent antifibrotique destiné à être utilisé selon l'une quelconque des revendications 1 à 5, dans lequel la biopsie myocardique est une biopsie myocardique du ventricule gauche.

7. Agent antifibrotique destiné à être utilisé selon l'une quelconque des revendications 1 à 6, dans lequel le pourcentage de fibrose myocardique est déterminé en plus ou à la place par tomographie par résonance magnétique (TRM) cardiaque, en particulier par imagerie par enrichissement tardif en gadolinium et/ou par cartographie T1.

8. Agent antifibrotique destiné à être utilisé selon l'une quelconque des revendications 1 à 7, dans lequel le patient présente une sténose aortique du sous-type PLF-LG AS ou du sous-type LEF-LG AS ;
en particulier dans lequel le patient présente une sténose aortique du sous-type PLF-LG AS.

9. Agent antifibrotique destiné à être utilisé selon l'une quelconque des revendications 1 à 8, dans lequel l'agent antifibrotique est une thérapie combinée avec (i) de la spironolactone et (ii) de l'hydralazine.

10. Agent antifibrotique destiné à être utilisé selon l'une quelconque des revendications 1 à 9, dans lequel l'hydralazine ou la dihydralazine est administrée à une concentration inférieure à celle utilisée pour le traitement de l'hypertension, en particulier dans lequel la concentration est choisie suffisamment faible pour présenter un effet déméthylant.

11. Agent antifibrotique destiné à être utilisé selon la revendication 9 ou la revendication 10, dans lequel l'hydralazine ou la dihydralazine est administrée aux métaboliseurs rapides à une dose comprise entre 2 x 15,1 mg par jour et 2 x 25 mg par jour et aux métaboliseurs lents à une dose comprise entre 2 x 5 mg par jour et 2 x 15 mg par jour.

12. Agent antifibrotique destiné à être utilisé selon l'une quelconque des revendications 9 à 11, dans lequel l'hydralazine ou la dihydralazine est administrée aux métaboliseurs rapides à un dosage de 2 x 25 mg par jour et aux métaboliseurs lents à un dosage de 2 x 12,5 mg par jour.

**Abb. 1**

**Abb. 2**

A

B

**Abb. 2 (fortgesetzt)**

C

D

**Abb. 3**

**Abb. 3 (fortgesetzt)**

**C**

**Abb. 4**

**A**

**B**

**Abb. 5**

Hazard Verhältnis

| Variable | Hazard Verhältnis (95%-KI) | p-Wert |
|---|---|---|
| Schlagvolumenindex (SVI) | 2,87 (0,09 - 90,4) | 0,549 |
| LV-EF (pro %-p. Abnahme) | 1,01 (0,91 - 1,1) | 0,845 |
| LVEDV (pro mL Zunahme) | 1,03 (1 - 1,1) | 0,02* |
| Weibliches Geschlecht | 14,06 (1,19 - 166) | 0,036* |
| Alter ≥ 79 Jahre (=Median) | 0,97 (0,83 - 1,1) | 0,684 |
| NHYA Klasse IV am Anfang | 1,34 (0,16 - 11,6) | 0,788 |
| Periphere Gefäßerkrankung | 41,25 (4,21 - 404) | 0,001** |
| GFR < 60 ml/min/1,72m² | 4,30 (0,48 - 38,9) | 0,194 |
| Diabetes | 3,79 (0,31 - 45) | 0,296 |
| Vorhofflimmern | 71,84 (5,61 - 919,6) | 0,001** |
| Myokardfibrose in LV Biopsien ≥11% | 27,41 (2,04 - 389) | 0,013* |
| AS Subtyp: NEF-HG AS | Referenz | |
| LEF-HG AS | 1,41 (0,02 - 82,1) | 0,868 |
| LEF-LG AS | 9,41 (0,15 - 602,2) | 0,291 |
| PFL-LG AS | 2,05 (0,09 - 48,5) | 0,656 |

Achse: 0,1   1   10   100   1000

#Ereignisse: 14 Globaler p-Wert (Log-Rank): 3,8707e-07

AIC: 81,47; Konkordanzindex: 0,95

**Abb. 6**

**Abb. 7**

Hazard Verhältnis

| | | | p-Wert |
|---|---|---|---|
| Schlagvolumenindex (SVI) < 35ml/l² | 1,73 (0,41 – 7,3) | | 0,458 |
| LV-EF (pro %-p. Abnahme) | 0,99 (0,92 – 1,1) | | 0,719 |
| LVEDV (pro mL Zunahme) | 1,01 (1 – 1) | | 0,155 |
| Weibliches Geschlecht | 1,79 (0,49 – 6,5) | | 0,377 |
| Alter ≥ 79 Jahre (=Median) | 0,95 (0,87 – 1) | | 0,269 |
| NHYA Klasse IV am Anfang | 0,70 (0,22 – 2,2) | | 0,551 |
| Periphere Gefäßerkrankung | 4,72 (1,54 – 14,5) | | 0,007** |
| GFR < 60 ml/min/1,72m² | 2,98 (0,91 – 9,8) | | 0,071 |
| Diabetes | 1,02 (0,33 – 3,2) | | 0,968 |
| Vorhofflimmern | 5,11 (1,53 – 17) | | 0,008** |
| Myokardfibrose in LV Biopsien ≥11% | 2,24 (0,71 – 7) | | 0,168 |
| AS Subtyp: NEF-HG AS | Referenz | | |
| LEF-HG AS | 0,54 (0,05 – 5,7) | | 0,607 |
| LEF-LG AS | 0,77 (0,09 – 6,6) | | 0,809 |
| PFL-LG AS | 0,68 (0,14 – 3,2) | | 0,623 |

#Ereignisse: 22 Globaler p-Wert (Log-Rank): < ,001

AIC: 178,02; Konkordanzindex: 0,79

0,1  0,2  0,5  1  2  5  10

EP 4 038 360 B1

Abb. 8

**Abb. 9**

**Abb. 10**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **BAUMGARTNER H** ; **FALK V** ; **BAX JJ** ; **DE BONIS M** ; **HAMM C** ; **HOLM PJ** ; **IUNG B, LANCEL** ; **LOTTI P** ; **LANSAC E** ; **RODRIGUEZ MUNOZ D**. ESC/EACTS Guidelines for the management of valvular heart disease. *Eur Heart J.*, 2017, vol. 38, 2739-2791
- **HEIN S** ; **ARNON E** ; **KOSTIN S** ; **SCHONBURG M** ; **ELSASSER A** ; **POLYAKOVA V** ; **BAUER EP** ; **KLOVEKORN WP** ; **SCHAPER J.** Progression from compensated hypertrophy to failure in the pressure-overloaded human heart: structural deterioration and compensatory mechanisms. *Circulation*, 2003, vol. 107, 984-91
- **KRAYENBUEHL HP** ; **HESS OM** ; **MONRAD ES** ; **SCHNEIDER J** ; **MALL G** ; **TURINA M.** Left ventricular myocardial structure in aortic valve disease before, intermediate, and late after aortic valve replacement. *Circulation*, 1989, vol. 79, 744-55
- **WEIDEMANN F** ; **HERRMANN S** ; **STORK S** ; **NIEMANN M** ; **FRANTZ S** ; **LANGE V** ; **BEER M** ; **GATTENLOHNER S** ; **VOELKER W** ; **ERTL G**. Impact of myocardial fibrosis in patients with symptomatic severe aortic stenosis. *Circulation*, 2009, vol. 120, 577-84
- **AZEVEDO CF** ; **NIGRI M** ; **HIGUCHI ML** ; **POMERANTZEFF PM** ; **SPINA GS** ; **SAMPAIO RO** ; **TARASOUTCHI F** ; **GRINBERG M** ; **ROCHITTE CE**. Prognostic significance of myocardial fibrosis quantification by histopathology and magnetic resonance imaging in patients with severe aortic valve disease. *J Am Coll Cardiol*, 2010, vol. 56, 278-87
- **KAPPETEIN AP** ; **HEAD SJ** ; **GENEREUX P** ; **PIAZZA N** ; **VAN MIEGHEM NM** ; **BLACKSTONE EH** ; **BROTT TG** ; **COHEN DJ, CUTLIP DE** ; **VAN ES GA** ; **HAHN RT**. Updated standardized endpoint definitions for transcatheter aortic valve implantation: the Valve Academic Research Consortium-2 consensus document. *Eur Heart J*, 2012, vol. 33, 2403-18
- **LANG RM** ; **BADANO LP** ; **MOR-AVI V** ; **AFILALO J** ; **ARMSTRONG A** ; **ERNANDE L** ; **FLACHSKAMPF FA** ; **FOSTER E** ; **GOLDSTEIN SA** ; **KUZNETSOVA T**. Recommendations for cardiac chamber quantification by echocardiography in adults: an update from the American Society of Echocardiography and the European Association of Cardiovascular Imaging. *European heart journal cardiovascular Imaging*, 2015, vol. 16, 233-70
- **MEWTON N** ; **LIU CY** ; **CROISILLE P** ; **BLUEMKE D** ; **LIMA JA**. Assessment of myocardial fibrosis with cardiovascular magnetic resonance. *J Am Coll Cardiol*, 2011, vol. 57, 891-903
- **TREIBEL TA** ; **LOPEZ B** ; **GONZALEZ A** ; **MENACHO K** ; **SCHOFIELD RS** ; **RAVASSA S** ; **FONTANA M** ; **WHITE SK** ; **DISALVO C** ; **ROBERTS N**. Reappraising myocardial fibrosis in severe aortic stenosis: an invasive and noninvasive study in 133 patients. *Eur Heart J*, 2018, vol. 39, 699-709
- **HERRMANN S** ; **STORK S** ; **NIEMANN M** ; **LANGE V** ; **STROTMANN JM** ; **FRANTZ S** ; **BEER M** ; **GATTENLOHNER S** ; **VOELKER W** ; **ERTL G**. Low-gradient aortic valve stenosis myocardial fibrosis and its influence on function and outcome. *J Am Coll Cardiol*, 2011, vol. 58, 402-12
- **MACK MJ** ; **LEON MB** ; **THOURANI VH** ; **MAKKAR R** ; **KODALI SK** ; **RUSSO M** ; **KAPADIA SR** ; **MALAISRIE SC** ; **COHEN DJ** ; **PIBAROT P**. Transcatheter Aortic-Valve Replacement with a Balloon-Expandable Valve in Low-Risk Patients. *The New England Journal of Medicine*, 2019, vol. 380, 1695-1705
- **MUSA TA** ; **TREIBEL TA** ; **VASSILIOU VS** ; **CAPTUR G** ; **SINGH A** ; **CHIN C** ; **DOBSON LE** ; **PICA S** ; **LOUDON M** ; **MALLEY T**. Myocardial Scar and Mortality in Severe Aortic Stenosis. *Circulation*, 2018, vol. 138, 1935-1947
- **TREIBEL TA** ; **KOZOR R** ; **SCHOFIELD R** ; **BENEDETTI G** ; **FONTANA M** ; **BHUVA AN** ; **SHEIKH A** ; **LOPEZ B** ; **GONZALEZ A** ; **MANISTY C**. Reverse Myocardial Remodeling Following Valve Replacement in Patients With Aortic Stenosis. *J. Am Coll Cardiol*, 2018, vol. 71, 860-871
- **CHEN H** ; **ZENG J** ; **LIU D** ; **YANG Q**. Prognostic value of late gadolinium enhancement on CMR in patients with severe aortic valve disease: a systematic review and metaanalysis. *Clinical radiology*, 2018, vol. 73, 983.e7-983.e14
- **LEE H** ; **PARK JB** ; **YOON YE** ; **PARK EA** ; **KIM HK** ; **LEE W** ; **KIM YJ** ; **CHO GY** ; **SOHN DW** ; **GREISER A**. Noncontrast Myocardial T1 Mapping by Cardiac Magnetic Resonance Predicts Outcome in Patients With Aortic Stenosis. *JACC Cardiovascular Imaging*, 2018, vol. 11, 974-983

- **BING R ; CAVALCANTE JL ; EVERETT RJ ; CLAVEL MA ; NEWBY DE ; DWECK MR**. Imaging and Impact of Myocardial Fibrosis in Aortic Stenosis. *JACC Cardiovascular Imaging*, 2019, vol. 12, 283-296

- **MONRAD ES ; HESS OM ; MURAKAMI T ; NONOGI H ; CORIN WJ ; KRAYENBUEHL HP**. Time course of regression of left ventricular hypertrophy after aortic valve replacement. *Circulation*, 1988, vol. 77, 1345-55

- **DOBSON LE ; MUSA TA ; UDDIN A ; FAIRBAIRN TA ; SWOBODA PP ; ERHAYIEM B ; FOLEY J ; GARG P ; HAAF P ; FENT GJ**. Acute Reverse Remodelling After Transcatheter Aortic Valve Implantation: A Link Between Myocardial Fibrosis and Left Ventricular Mass Regression. *The Canadian journal of cardiology*, 2016, vol. 32, 1411-1418

- **LAMB HJ ; BEYERBACHT HP ; DE ROOS A ; VAN DER LAARSE A ; VLIEGEN HW ; LEUJES F, BAX JJ ; VAN DER WALL EE**. Left ventricular remodeling early after aortic valve replacement: differential effects on diastolic function in aortic valve stenosis and aortic regurgitation. *J Am Coll Cardiol*, 2002, vol. 40, 2182-8

- **ROST C ; KORDER S ; WASMEIER G ; WU M ; KLINGHAMMER L ; FLACHSKAMPF FA ; DANIEL WG ; VOIGT JU**. Sequential changes in myocardial function after valve replacement for aortic stenosis by speckle tracking echocardiography. *European journal of echocardiography : the journal of the Working Group on Echocardiography of the European Society of Cardiology*, 2010, vol. 11, 584-9

- **DWECK MR ; JOSHI S ; MURIGU T ; ALPEN-DURADA F ; JABBOUR A ; MELINA G ; BANYA W ; GULATI A ; ROUSSIN I ; RAZA S**. Midwall fibrosis is an independent predictor of mortality in patients with aortic stenosis. *J Am Coll Cardiol*, 2011, vol. 58, 1271-9

- **FOPPL M ; HOFFMANN A ; AMANN FW ; ROTH J ; STULZ P ; HASSE J ; GRADEL E ; BURCKHARDT D**. Sudden cardiac death after aortic valve surgery: incidence and concomitant factors. *Clinical cardiology*, 1989, vol. 12, 202-7

- **RAVASSA S ; TRIPPEL T ; BACH D ; BACHRAN D ; GONZÁLEZ A ; LÓPEZ B ; WACHTER R ; HASENFUSS G ; DELLES C ; DOMINICZAK AF**. Biomarker based phenotyping of myocardial fibrosis identifies patients with heart failure with preserved ejection fraction resistant to the beneficial effects of spironolactone: results from the Aldo-DHF trial. *Eur J Heart Fail*, 2018, vol. 20, 1290-1299

- **TAMPE B ; TAMPE D ; ZEISBERG EM ; MÜLLER GA ; BECHTEL-WALZ W ; KOZIOLEK M ; KALLURI R ; ZEISBERG M**. Induction of Tet3-dependent epigenetic remodeling by lowdose hydralazine attenuates progression of chronic kidney disease. *EBioMedicine*, 2015, vol. 2, 19-36

- **TIMBRELL JA ; HARLAND SJ ; FACCHINI V**. Polymorphic acetylation of hydralazine. *Clin. Pharmacol Ther.*, September 1980, vol. 28 (3), 350-355

- **RUIZ JD ; MARTINEZ C ; ANDERSON K ; GROSS M ; LANG NP ; GARCIA-MARTIN E ; AGUNDEZ JAG**. The Differential Effect of NAT2 Variant Alleles Permits Refinement in Phenotype Inference and Identifies a Very Slow Acetylation Genotype. *PLOS ONE*, 2012, vol. 7 (9), e44629

- **GARCES-EISELE SJ ; CEDILLO-CAVALLO B ; REYES-NUNEZ V ; ESTRADA-MARTIN L ; VAZQUEZ-PEREZ R ; JUAREZ-CALDERON M ; GUZMAN-GARCIA MO ; DUENAS-GONZALEZ A ; RUIZ-ARGÜELLES A**. Genetic selection of volunzteers and concomitant dose adjustment leads to comparable hydralazine/valproate exposure. *Journal of Clinical Pharmacy and Therapeutics*, 2014

- **SAWHNEY N ; HASSANKHANI A ; GREENBERG BH**. Calcific Aortic Stenosis in the Elderly: A Brief Overview. *The American Journal of Geriatric Cardiology*, 2003, vol. 12 (3), 178-182

- **MANGLA A ; GUPTA S**. Vascular complications post-transcatheter aortic valve procedures. *Indian Heart Journal*, 2016, vol. 68 (5), 724-731

- **LACOLLEY P ; SAFAR ME ; LUCET B ; LEDUDAL K ; LABAT C ; BENETOS A**. Prevention of Aortic and Cardiac Fibrosis by Spironolactone in Old Normotensive Rats. *J Am Coll Cardiol*, 2001, vol. 37 (2), 662-667

- **CEZAR MDM ; DAMATTO RL ; PAGAN LU ; LIMA ARR ; MARTINEZ PF ; BONOMO C ; ROSA CM ; CAMPOS DHS ; CICOGNA AC ; GOMES MJ**. Early Spironolactone Treatment Attenuates Heart Failure Development by Improving Myocardial Function and Reducing Fibrosis in Spontaneously Hypertensive Rats. *Cell Physiol Biochem*, 2015, vol. 36, 1453-1466

- **PONIKOWSKI et al.** ESC Guidelines for the diagnosis and treatment of acute and chronic heart failure. *Eur Heart J*, 2016, vol. 2016, ehw128

- **XU X ; TAN X ; TAMPE B ; NYAMSUREN G ; LIU X ; MAIER LS ; SOSSALLA S ; KALLURI R ; ZEISBERG M ; HASENFUSS G**. Epigenetic balance of aberrant Rasal1 promoter methylation and hydroxymethylation regulates cardiac fibrosis. *Cardiovascular Res*, 2015, vol. 105, 279-291

- **ZEISBERG EM ; ZEISBERG M**. A Rationale for Epigenetic Repurposing of Hydralazine in Chronic Heart and Kidney Failure. *J Clin Epigenet*, 2016, vol. 2 (1), 3

- **PULS M** ; **BEUTHNER BE** ; **TOPCI R** ; **VOGELGE-SANG A** ; **BLECKMANN A** ; **SITTE M** ; **LANGE T** ; **BACKHAUS SJ** ; **SCHUSTER A** ; **SEIDLER T**. Impact ofmyocardial fibrosis on left ventricular remodelling, recovery, and outcome after transcatheter aortic valve implantation in different haemodynamic subtypes of severe aortic stenosis. *Eur Heart J*, 2020, vol. 41, 1903-1914
- **MATSUI Y** ; **JIA N** ; **OKAMOTO H** ; **KON S** ; **ONOZUKA H** ; **AKINO M** ; **LIU L** ; **MORIMOTO J** ; **RITTLING SR** ; **DENHARDT D et al.** Role of osteopontin in cardiac fibrosis and remodeling in angiotensin II-induced cardiac hypertrophy. *Hypertension*, 2004, vol. 43, 1195-1201
- **SCHELLINGS MWM** ; **VANHOUTTE D** ; **ALMEN GC VAN** ; **SWINNEN M** ; **LEENDERS JJG** ; **KUBBEN N** ; **LEEUWEN REW VAN** ; **HOFSTRA L** ; **HEYMANS S** ; **PINTO YM**. Syndecan-1 Amplifies Angiotensin II-Induced Cardiac Fibrosis. *Hypertension*, 2010, vol. 55, 249-256
- **WEBER M** ; **DAVIES JJ** ; **WITTIG D** ; **OAKELEY EJ** ; **HAASE M** ; **LAM WL** ; **SCHÜBELER D**. *Chromosome-wide and promoter-specific analyses identify sites of differential DNA methylation in normal and transformed human cells*, 2005